# EUROPEAN PATENT APPLICATION

(11) **EP 3 954 776 A1**
(43) Date of publication of application: **16.02.2022**
(21) Application number: 20794478.6
(22) Date of filing: 10.03.2020
(51) Int. Cl.: C12N 15/77, C12N 9/06, C12N 9/10, C12N 9/18, C07K 14/34, C12P 13/24

(54) **MICROORGANISM WITH ENHANCED L-HISTIDINE PRODUCTION CAPACITY AND METHOD FOR PRODUCING HISTIDINE BY USING SAME**

(30) Priority: 22.04.2019 KR 20190046934
(71) Applicant: CJ Cheiljedang Corporation, Seoul 04560 (KR)
(72) Inventor: HUH, Lan, Seoul 04560 (KR); KWON, Nara, Seoul 04560 (KR); SEO, Chang Il, Seoul 04560 (KR)
(74) Representative: Icosa
(86) International application number: PCT/KR2020/003317
(87) International publication number: WO 2020/218736

(57) **Abstract**

Provided are a microorganism having an enhanced L-histidine producing ability and a method of producing histidine using the same.

## Description

### BACKGROUND OF THE INVENTION

### 1. Field of the Invention

The present disclosure relates to a microorganism having an enhanced L-histidine producing ability and a method of producing histidine using the same.

### 2. Description of the Related Art

L-Histidine is one of the 20 standard amino acids, the majority of which, from a nutritional point of view, are not required for adults, but it is classified as an essential amino acid for growing children. Additionally, L-histidine is involved in important physiological processes such as antioxidation, immune regulation, *etc.,* and thus is used in the medical industry, such as an agent for treating gastric ulcer, a raw material for a cardiovascular agent, amino acid sap, *etc.*

Histidine is mainly found in hemoglobin, and is primarily produced by a protein hydrolysis extraction method using blood meal as a raw material. However, it has disadvantages such as low efficiency, environmental pollution, *etc.* On the other hand, L-histidine can be produced through microbial fermentation, but large-scale industrialization has not yet been accomplished. This is because the biosynthesis of L-histidine competes with a nucleotide synthesis precursor, *i.e.,* PRPP, and has a complicated biosynthetic process and regulatory mechanism requiring high energy.

The L-histidine producing ability of a microorganism used in a fermentation method has been improved by a mutagenic and mutant selection method and a method of regulating the metabolism of a strain through genetic modification. Recently, the production of histidine using microorganisms has been known to be accomplished by biosynthesis from PRPP via several steps. However, an ATP phosphoribosyl transferase, which is the first enzyme involved in the biosynthesis of histidine, has feedback inhibition by the final product, *i.e.,* histidine, or a derivative thereof, and this is a problem in the industrial mass production of L-histidine (International Patent Publication No. WO 2014-029376). Due to this complicated biosynthetic process and regulatory mechanism, an approach from various perspectives related to microbial metabolism is necessary in order to produce L-histidine through microbial culture.

To develop a microorganism which may utilize glycine by importing the glycine exported out of cells, the present inventors introduced a glycine transporter cycA derived from *Corynebacterium ammoniagenes,* and as result, they have completed a microorganism producing L-histidine with a high yield.

### SUMMARY OF THE INVENTION

An object of the present disclosure is to provide a microorganism of the genus *Corynebacterium* for producing L-histidine, the microorganism having an enhanced glycine transporter activity.

Another object of the present disclosure is to provide a composition for producing L-histidine, the composition including the microorganism of the present disclosure.

Still another object of the present disclosure is to provide a method of producing L-histidine, the method including the step of culturing the microorganism of the present disclosure.

Still another object of the present disclosure is to provide use of the microorganism of the genus *Corynebacterium* having an enhanced glycine transporter activity in the production of L-histidine.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

One aspect of the present disclosure provides a microorganism of the genus *Corynebacterium* for producing L-histidine, the microorganism having an enhanced glycine transporter activity.

As used herein, the term "glycine transporter" may include any protein capable of introducing glycine into cells, and it may be specifically D-serine/D-alanine/glycine transporter. The glycine transporter may be interchangeably used with D-serine/D-alanine/glycine transporter or a protein for glycine uptake.

The "D-serine/D-alanine/glycine transporter" is a protein that may be involved in the transport of all of serine, alanine, and glycine, and information thereof may be obtained by searching for the D-serine/D-alanine/glycine transporter sequence in a known database such as NCBI Genbank, *etc.* The transporter may be specifically CycA or AapA, and more specifically a CycA protein, but is not limited thereto.

As used herein, the term "CycA protein" refers to a protein involved in serine, alanine, and glycine uptake. The CycA protein is encoded by a cycA gene, and the cycA gene is known to exist in microorganisms, such as *Escherichia coli, Klebsiella pneumoniae, Mycobacterium bovis, Salmonella enterica, Erwinia amylovora, Corynebacterium ammoniagenes, etc.*

With respect to the objects of the present disclosure, the CycA protein of the present disclosure may include any protein as long as it may enhance the histidine producing ability. Specifically, the CycA protein may be derived from a microorganism of the genus *Corynebacterium* or the genus *Escherichia,* and more specifically *Corynebacterium ammoniagenes,* but is not limited thereto. *Corynebacterium ammoniagenes* is the same species as *Brevibacterium ammoniagenes,* and has been classified in the same taxon as *Corynebacterium stationis* and *Brevibacterium stationis (*International Journal of Systematic and Evolutionary Microbiology 60:874-879). Additionally, *Brevibacterium ammoniagenes* has been renamed as *Corynebacterium stationis.*

Accordingly, as used herein, the terms *Corynebacterium ammoniagenes, Brevibacterium ammoniagenes, Corynebacterium stationis,* and *Brevibacterium stationis* may be used interchangeably.

The CycA protein of the present disclosure may include an amino acid sequence of SEQ ID NO: 1 or an amino acid sequence having 70% or more homology or identity thereto.

Specifically, the CycA protein may include an amino acid sequence of SEQ ID NO: 1 or an amino acid sequence having at least 70%, 75%, 80%, 85%, 86%, 87%, 88%, 89%, 90%, 91%, 92%, 93%, 94%, 95%, 96%, 97%, 98%, or 99% homology or identity to the amino acid sequence of SEQ ID NO: 1. Additionally, it is apparent that any amino acid sequence in which part of the sequence is deleted, modified, substituted, or added may also fall within the scope of the present disclosure, as long as the amino acid sequence has such a homology or identity and exhibits efficacy corresponding to that of the above protein.

Further, a probe that may be prepared from a known gene sequence, for example, any polypeptide encoded by a polynucleotide which may hybridize with a sequence complementary to all or part of a nucleotide sequence under stringent conditions to encode the polypeptide, may include polypeptides having the serine, alanine, and glycine uptake activity without limitation.

In other words, although it is described as "a protein or polypeptide including an amino acid sequence described by a specific sequence number", "a protein or polypeptide consisting of an amino acid sequence described by a specific sequence number", or a "protein or polypeptide having an amino acid sequence described by a specific sequence number" in the present disclosure, it is apparent that any protein having an amino acid sequence in which part of the sequence is deleted, modified, substituted, conservatively substituted, or added may be used in the present disclosure as long as it has the same or corresponding activity as the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, a silent mutation thereof, or a conservative substitution.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan.

As used herein, the term "polynucleotide" has a meaning which collectively includes DNA or RNA molecules. Nucleotides, which are the basic structural units of the polynucleotides, include not only natural nucleotides but also modified analogs thereof in which sugar or base sites are modified (see Scheit, Nucleotide Analogs, John Wiley, New York (1980); Uhlman and Peyman, Chemical Reviews, 90:543-584 (1990)).

The polynucleotide may be a polynucleotide encoding the CycA protein of the present disclosure, or may be a polynucleotide encoding a polypeptide having at least 70%, 75%, 80%, 85%, 86%, 87%, 88%, 89%, 90%, 91%, 92%, 93%, 94%, 95%, 96%, 97%, 98%, or 99% homology or identity to the CycA protein of the present disclosure. Specifically, for example, the polynucleotide encoding the protein including the amino acid sequence of SEQ ID NO: 1 or the amino acid sequence having 70% or more homology or identity to SEQ ID NO: 1 may be a polynucleotide including a polynucleotide sequence of SEQ ID NO: 2 or having at least 70%, 75%, 80%, 85%, 86%, 87%, 88%, 89%, 90%, 91%, 92%, 93%, 94%, 95%, 96%, 97%, 98%, or 99% homology or identity to the polynucleotide sequence of SEQ ID NO: 2.

Additionally, based on codon degeneracy, it is apparent that polynucleotides which may be translated into proteins including the amino acid sequence of SEQ ID NO: 1 or an amino acid sequence having 70% or more identity to SEQ ID NO: 1, or proteins having a homology or identity thereto may also be included. Additionally, the polynucleotide of the present disclosure may include a probe that may be prepared from a known gene sequence, for example, any polynucleotide sequence which may hybridize with a sequence complementary to all or part of the polynucleotide sequence under stringent conditions to encode proteins including the amino acid sequence having 70% or more identity to the amino acid sequence of SEQ ID NO: 1, without limitation. The "stringent conditions" refer to conditions under which specific hybridization between polynucleotides is allowed. Such conditions are specifically described in the literature *(e.g.,* J. Sambrook et al., Molecular Cloning, A Laboratory Manual, 2nd Edition, Cold Spring Harbor Laboratory Press, Cold Spring Harbor, New York, 1989; F. M. Ausubel et al., Current Protocols in Molecular Biology, John Wiley & Sons, Inc., New York). For example, the stringent conditions may include conditions under which genes having a high homology or identity of 70% or higher, 80% or higher, specifically 85% or higher, specifically 90% or higher, more specifically 95% or higher, much more specifically 97% or higher, and still much more specifically 99% or higher are hybridized with each other, and genes having a homology or identity lower than the above homologies or identities are not hybridized with each other, or washing conditions of Southern hybridization, that is, washing once, specifically twice or three times at a salt concentration and a temperature corresponding to 60°C, 1×SSC, 0.1% SDS, specifically 60°C, 0.1× SSC, 0.1% SDS, and more specifically 68°C, 0.1×SSC, 0.1% SDS. Hybridization requires that two polynucleotides have complementary sequences, although mismatches between bases are possible depending on the stringency of the hybridization. The term "complementary" is used to describe the relationship between nucleotide bases that may hybridize with each other. For example, with respect to DNA, adenosine is complementary to thymine, and cytosine is complementary to guanine. Therefore, the polynucleotide of the present disclosure may include isolated polynucleotide fragments complementary to the entire sequence as well as polynucleotide sequences substantially similar thereto.

Specifically, the polynucleotides having a homology or identity may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

As used herein, the term "homology" or "identity" refers to a degree of relatedness between two given amino acid sequences or nucleotide sequences, and may be expressed as a percentage. The terms homology and identity may be often used interchangeably with each other. The sequence homology or identity of conserved polynucleotide or polypeptide sequences may be determined by standard alignment algorithms and may be used with a default gap penalty established by the program being used. Substantially, homologous or identical sequences are generally expected to hybridize to all or at least about 50%, 60%, 70%, 80%, or 90% of the entire length of the sequences under moderate or highly stringent conditions. Polynucleotides that contain degenerate codons instead of codons in hybridizing polynucleotides are also considered.

The homology or identity of the polypeptide or polynucleotide sequences may be determined by, for example, the BLAST algorithm according to the literature (see Karlin and Altschul, Pro. Natl. Acad. Sci. USA, 90, 5873 (1993)), or FASTA by Pearson (see Methods Enzymol., 183, 63, 1990). Based on the algorithm BLAST, a program referred to as BLASTN or BLASTX has been developed (see http://www.ncbi.nlm.nih.gov). Further, whether any amino acid or polynucleotide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art *(e.g.,* J. Sambrook et al., Molecular Cloning, A Laboratory Manual, 2nd Edition, Cold Spring Harbor Laboratory Press, Cold Spring Harbor, New York, 1989; F. M. Ausubel et al., Current Protocols in Molecular Biology, John Wiley & Sons, Inc., New York).

As used herein, the term "enhancement of protein activity" means that the activity is enhanced as compared to the endogenous activity possessed by a microorganism or the activity before transformation. The enhancement of activity may include both introducing a foreign protein and enhancing the activity of an endogenous protein. That is, it includes introducing a foreign protein into a microorganism having intrinsic activity of a specific protein, and introducing the protein into a microorganism having no intrinsic activity. The "introduction of the protein" means that activity of a specific protein is introduced into a microorganism such that the protein activity is modified for expression. It may also be expressed as the enhancement of the activity of the corresponding protein.

As used herein, the term "endogenous" refers to a state originally possessed by a parent strain prior to transformation, when the traits of the microorganism are altered by way of genetic modification due to natural or artificial factors.

In the present disclosure, the enhancement of activity may be performed by way of the following methods of:
1) increasing the copy number of the polynucleotide encoding the protein;
2) modifying an expression regulatory sequence such that the expression of the polynucleotide is increased;
3) modifying the polynucleotide sequence on a chromosome such that the activity of the protein is enhanced;
4) introducing a foreign polynucleotide exhibiting the activity of the protein or a modified polynucleotide in which the codons of the above polynucleotide have been optimized; or
5) modification to enhance the activity by way of a combination of the above methods, but the method is not limited thereto.
1) The increasing of the copy number of the polynucleotide may be performed in a form in which the polynucleotide is operably linked to a vector, or by inserting into a chromosome of a host cell, but is not particularly limited thereto. Specifically, it may be performed by operably linking the polynucleotide encoding the protein of the present disclosure to a vector which may replicate and function regardless of the host cell, and then introducing the same into the host cell. Alternatively, it may be performed by way of a method of increasing the copy number of the polynucleotide in the chromosome of the host cell by operably linking the polynucleotide to a vector which may insert the polynucleotide into the chromosome of the host cell, and introducing the same into the host cell.

Next, 2) the modification of an expression regulatory sequence such that the expression of the polynucleotide is increased may be performed by inducing a modification in the sequence through deletion, insertion, or non-conservative or conservative substitution of a nucleic acid sequence, or a combination thereof so as to further enhance the activity of the expression regulatory sequence, or by replacing with a nucleic acid sequence having a stronger activity, but is not particularly limited thereto. The expression regulatory sequence may include a promoter, an operator sequence, a sequence encoding a ribosome binding domain, a sequence regulating the termination of transcription and translation, *etc.,* but is not particularly limited thereto.

A strong heterologous promoter may be linked to the upstream region of the expression unit of the polynucleotide instead of the original promoter. Examples of the strong promoter include CJ7 promoter (Korean Patent No. 0620092 and WO 2006/065095), lysCP1 promoter (WO 2009/096689), EF-Tu promoter, groEL promoter, aceA or aceB promoter, *etc.,* but the strong promoter is not limited thereto. Further, 3) the modification of the polynucleotide sequence on a chromosome may be performed by inducing a modification in the expression regulatory sequence through deletion, insertion, or non-conservative or conservative substitution of a nucleic acid sequence, or a combination thereof so as to further enhance the activity of the polynucleotide sequence, or by replacing the polynucleotide sequence with a polynucleotide sequence modified to have a stronger activity, but is not particularly limited thereto.

Additionally, 4) the introduction a foreign polynucleotide sequence may be performed by introducing, into a host cell, a foreign polynucleotide encoding a protein that exhibits an activity identical or similar to that of the protein above, or a modified polynucleotide in which the codons of the foreign polynucleotide have been optimized. The foreign polynucleotide may be used without limitation to its origin or sequence as long as it exhibits an activity identical or similar to that of the protein. Further, the foreign polynucleotide may be introduced into a host cell after optimization of its codons so as to achieve the optimized transcription and translation in the host cell. The introduction may be performed by those skilled in the art by appropriately selecting a known transformation method, and a protein may be produced as the introduced polynucleotides are expressed in the host cell, thereby increasing its activity.

Finally, 5) the method of modification to enhance the activity by way of a combination of 1) to 4) may be performed by way of a combined application of one or more of the following methods: increasing the copy number of the polynucleotide encoding the protein; modifying an expression regulatory sequence such that the expression of the polynucleotide is increased; modifying the polynucleotide sequence on a chromosome; and modifying a foreign polynucleotide exhibiting the activity of the protein or a codon-optimized modified polynucleotide thereof.

As used herein, the term "vector" refers to a DNA construct containing the polynucleotide sequence encoding the target protein, which is operably linked to a suitable regulatory sequence such that the target protein may be expressed in an appropriate host. The regulatory sequence includes a promoter capable of initiating transcription, any operator sequence for the control of the transcription, a sequence encoding an appropriate mRNA ribosome binding domain, and a sequence regulating the termination of transcription and translation. After being transformed into a suitable host cell, the vector may be replicated or function irrespective of the host genome, and may be integrated into the host genome itself. For example, a polynucleotide encoding a target protein in the chromosome may be replaced with a modified polynucleotide through a vector for chromosomal insertion. The insertion of the polynucleotide into the chromosome may be performed by way of any method known in the art, for example, homologous recombination, but is not limited thereto.

The vector used in the present disclosure is not particularly limited, and any vector known in the art may be used. Examples of commonly used vectors may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, Charon21A, *etc.* may be used, and as a plasmid vector, those based on pBR, pUC, pBluescriptII, pGEM, pTZ, pCL, pET, *etc.* may be used. Specifically, the vectors pDZ, pACYC177, pACYC184, pCL, pECCG117, pUC19, pBR322, pMW118, pCC1BAC, *etc.* may be used.

As used herein, the term "transformation" refers to a process of introducing a vector including a polynucleotide encoding a target polypeptide into a host cell, thereby enabling expression of the polypeptide encoded by the polynucleotide in the host cell. As long as the transformed polynucleotide may be expressed in the host cell, it does not matter whether it is inserted into the chromosome of a host cell and located therein or located outside the chromosome, and both cases may be included. Additionally, the polynucleotide includes DNA and RNA which encode the target protein. The polynucleotide may be introduced in any form as long as it may be introduced into a host cell and expressed therein. For example, the polynucleotide may be introduced into a host cell in the form of an expression cassette, which is a gene construct including all elements necessary for self-expression. The expression cassette may generally include a promoter operably linked to the polynucleotide, a transcription terminator, a ribosome binding domain, and a translation terminator. The expression cassette may be in the form of an expression vector capable of self-replication. Additionally, the polynucleotide may be introduced as it is into a host cell and operably linked to a sequence necessary for its expression in the host cell, but is not limited thereto.

Further, as used herein, the term "operably linked" refers to a functional linkage between the above gene sequence and a promoter sequence which initiates and mediates the transcription of the polynucleotide encoding the target protein of the present disclosure.

The method of transforming the vector of the present disclosure includes any method of introducing a nucleic acid into a cell, and may be performed by selecting a suitable standard technique as known in the art depending on the host cell. For example, the method may include electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but is not limited thereto.

As used herein, the term "microorganism for producing L-histidine" includes all wild-type microorganisms, or naturally or artificially genetically modified microorganisms, and it may refer to a microorganism naturally having the L-histidine producing ability, or a microorganism prepared by imparting the L-histidine producing ability to a parent strain without the L-histidine producing ability. Additionally, it may be a microorganism in which a particular mechanism is weakened or enhanced due to insertion of a foreign gene, or enhancement or inactivation of the activity of an endogenous gene, and it may be a microorganism in which genetic mutation occurs or activity is enhanced for the production of the desired L-histidine.

For example, the microorganism for producing L-histidine may be a microorganism having an enhanced glycine transporter activity. Alternatively, it may be a microorganism in which feedback of an enzyme on the histidine biosynthesis pathway is inhibited, a microorganism that produces histidine by enhancing or inhibiting an enzyme involved in the histidine biosynthesis pathway, or a microorganism that produces histidine by inactivating the activity of an enzyme or protein that does not affect histidine biosynthesis, thereby facilitating the metabolism of the histidine biosynthesis pathway.

Specifically, it may be a microorganism in which the activity of CycA protein is enhanced, or additionally, the feedback inhibition on the histidine biosynthesis pathway is inhibited by modifying a HisG polypeptide, or expression of one or more of genes encoding the enzyme group in the histidine biosynthesis pathway, including hisE, hisG, hisA, hisF, hisI, hisD, hisC, hisB, and hisN, is enhanced. Further, the microorganism may be a microorganism in which the enzyme in the histidine degradation pathway is inactivated, the activity of an intermediate, a cofactor, or a protein or enzyme on a pathway that consumes an energy source on the histidine biosynthesis pathway is inactivated, or a protein importing the target histidine is inactivated. For example, the microorganism may be a microorganism in which gamma-aminobutyrate permease (NCgl1 108) is inactivated.

Additionally, the microorganism may be a microorganism in which activity of a protein or an enzyme not involved in the growth or histidine biosynthesis of the microorganism is inactivated. More specifically, the microorganism may be a microorganism in which activity of formyltetrahydrofolate deformylase (PurU) or transposase (NCgl2131) not involved in the growth or L-histidine biosynthesis of the microorganism is attenuated.

As used herein, the term "inactivation of protein activity" means that the enzyme or protein is not expressed, or it has no activity thereof or has decreased activity even though expressed, as compared to a natural wild-type strain, a parent strain, or a strain in which the corresponding protein is not modified. In this regard, the decrease is a comprehensive concept including the case where the protein activity itself is decreased as compared with the activity of the protein originally possessed by a microorganism due to the mutation of the gene encoding the protein, modification of the expression regulatory sequence, or deletion in a part or all of genes, *etc.;* the case where the overall level of intracellular protein activity is decreased as compared with that of a natural strain or a strain before modification due to the inhibition of expression of the gene encoding the protein or the inhibition of translation; and a combination thereof. In the present disclosure, the inactivation may be achieved by applying various methods well known in the art. Examples of the methods may include 1) a method of deleting a part or all of the gene encoding the protein; 2) a method of modifying the expression regulatory sequence such that the expression of the gene encoding the protein is decreased; 3) a method of modifying the gene sequence encoding the protein such that the protein activity is removed or weakened; 4) a method of introducing an antisense oligonucleotide (e.g., antisense RNA) that binds complementarily to the transcript of the gene encoding the protein; 5) a method of adding a complementary sequence to the Shine-Dalgarno sequence upstream of the Shine-Dalgarno sequence of the gene encoding the protein to form a secondary structure, thereby inhibiting the ribosomal attachment; and 6) a reverse transcription engineering (RTE) method of adding a promoter at the 3' terminus of an open reading frame (ORF) of the polynucleotide sequence of the gene encoding the protein so as to be reversely transcribed; and a combination thereof, but is not particularly limited thereto.

However, this is merely an example, and the method is not limited thereto. Additionally, it may be a microorganism, in which the expression of genes encoding enzymes of various known L-histidine biosynthesis pathways is enhanced, enzymes on degradation pathways are inactivated, the activity of an intermediate, a cofactor, or an enzyme on a pathway that consumes an energy source on the histidine biosynthesis pathway is inactivated. The microorganism for producing L-histidine may be prepared by applying various known methods.

With respect to the objects of the present disclosure, the microorganism of the present disclosure may be any microorganism as long as it includes the glycine transporter and is capable of producing L-histidine.

As used herein, the term "microorganism capable of producing L-histidine" may be interchangeably used with "microorganism producing L-histidine", "microorganism having an L-histidine producing ability", and "microorganism for producing L-histidine".

The microorganism producing histidine of the present disclosure may be a microorganism in which the activity of the glycine cleavage protein is further enhanced. The "microorganism producing histidine" and "enhancement of protein activity" are the same as described above.

As used herein, the term "glycine cleavage protein" is a protein that is directly or indirectly involved in the glycine cleavage pathway, and may be used to mean each protein constituting the "glycine cleavage system (GCV)", or the complex of the proteins, or the glycine cleavage system itself.

Specifically, the glycine cleavage protein may be any one or more selected from the group consisting of T-protein (GcvT), P-protein (GcvP), L-protein (GcvL), H-protein (GcvH) that constitute the glycine cleavage system, and LipB or LipA, which are coenzymes of the glycine cleavage system, but is not limited thereto (John E. Cronan, Microbiology and Molecular Biology Reviews, 13 April 2016). The glycine cleavage protein may be derived from a microorganism of the genus *Corynebacterium,* specifically *Corynebacterium ammoniagenes,* but is not limited thereto. The GcvP protein may include an amino acid sequence of SEQ ID NO: 26, the GcvT protein may include an amino acid sequence of SEQ ID NO: 27, the GcvH protein may include an amino acid sequence of SEQ ID NO: 28, the LipA protein may include an amino acid sequence of SEQ ID NO: 29, and the LipB protein may include an amino acid sequence of SEQ ID NO: 30, or each may include an amino acid sequence having 70% or more homology to the respective amino acid sequence, but is not limited thereto. Specifically, the GcvP protein may include the amino acid sequence of SEQ ID NO: 26, or an amino acid sequence having at least 70%, 75%, 80%, 85%, 86%, 87%, 88%, 89%, 90%, 91%, 92%, 93%, 94%, 95%, 96%, 97%, 98%, or 99% homology or identity to the amino acid sequence of SEQ ID NO: 26. The description of homology or identity is the same for GcvT, GcvH, LipA, and LipB. Additionally, it is apparent that any protein having an amino acid sequence in which part of the amino acid sequence is deleted, modified, substituted, or added may also fall within the scope of the present disclosure as long as the amino acid has such a homology or identity and exhibits efficacy corresponding to that of the above protein.

Further, a probe that may be prepared from a known gene sequence, for example, any polypeptide having a glycine cleavage activity as a polypeptide encoded by a polynucleotide which may hybridize with a sequence complementary to all or part of the nucleotide sequence encoding the polypeptide under stringent conditions, may be included without limitation.

The homology or identity are as described above.

As used herein, the term "microorganism of the genus *Corynebacterium* for producing L-histidine" may refer to a microorganism that produces L-histidine and belongs to the genus *Corynebacterium.* The microorganism producing L-histidine is the same as described above. Specifically, in the present disclosure, the microorganism of the genus *Corynebacterium* having an L-histidine producing ability may refer to a microorganism of the genus *Corynebacterium* in which the activity of the glycine transporter of the present disclosure is enhanced, or which has been transformed with a vector containing the gene encoding the glycine transporter to have an enhanced L-histidine producing ability. Alternatively, it may refer to a microorganism of the genus *Corynebacterium* in which the activity of the glycine cleavage protein is further enhanced, or which has been transformed with a vector containing the gene encoding the glycine cleavage protein to have an enhanced L-histidine producing ability. The "microorganism of the genus *Corynebacterium* having an enhanced L-histidine producing ability" may refer to a microorganism in which the L-histidine producing ability is improved as compared with a parent strain before transformation or a non-modified microorganism. The 'non-modified microorganism' may refer to a natural strain of the genus *Corynebacterium* itself, a microorganism not containing the gene encoding the glycine transporter, or a microorganism that has not been transformed with a vector containing the gene encoding the glycine transporter.

As used herein, the term "microorganism of the genus *Corynebacterium"* may include all microorganisms of the genus *Corynebacterium.* Specifically, it may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically *Corynebacterium glutamicum.*

Another aspect of the present disclosure provides a composition for producing L-histidine, the composition including the microorganism for producing L-histidine of the present disclosure.

The composition for producing L-histidine may refer to a composition capable of producing L-histidine by the microorganism for producing L-histidine of the present disclosure. The composition may include the microorganism for producing L-histidine, and may include an additional composition capable of producing histidine using the strain without limitation. The additional component capable of producing histidine may further include, for example, any suitable excipient commonly used in a composition for fermentation, or components of a medium. Such excipients may be, for example, preservatives, wetting agents, dispersing agents, suspending agents, buffers, stabilizing agents, isotonic agents, *etc.,* but are not limited thereto.

Still another aspect of the present disclosure provides use of the microorganism of the genus *Corynebacterium* having an enhanced glycine transporter activity in the production of L-histidine.

The "glycine transporter", "enhancement of activity", or "microorganism of the genus *Corynebacterium"* are as described above.

Still another aspect of the present disclosure provides a method of producing L-histidine, the method including the step of culturing the microorganism.

The medium and other culture conditions used for culturing the microorganism of the present disclosure may be any medium commonly used for culturing the microorganism of the genus *Corynebacterium* without any particular limitation. Specifically, the microorganism of the present disclosure may be cultured under aerobic or anaerobic conditions in a common medium containing an appropriate carbon source, nitrogen source, phosphorus source, inorganic compound, amino acid, and/or vitamin, *etc.,* while adjusting temperature, pH, *etc.*

In the present disclosure, the carbon source may include carbohydrates, such as glucose, fructose, sucrose, maltose, *etc.;* alcohols, such as sugar alcohols, glycerol, *etc.;* fatty acids, such as palmitic acid, stearic acid, linoleic acid, *etc.;* organic acids, such as pyruvic acid, lactic acid, acetic acid, citric acid, *etc.;* amino acids, such as glutamic acid, methionine, lysine, *etc.,* but is not limited thereto. Additionally, the carbon source may include natural organic nutrients such as starch hydrolysate, molasses, blackstrap molasses, rice bran, cassava, sugar cane molasses, corn steep liquor, *etc.,* and carbohydrates such as sterilized pretreated molasses *(i.e.,* molasses converted to reducing sugar) may be used. In addition, various other carbon sources in an appropriate amount may be used without limitation. These carbon sources may be used alone or in combination of two or more kinds thereof.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition products thereof, defatted soybean cake or decomposition products thereof, *etc.* These nitrogen sources may be used alone or in combination of two or more kinds thereof, but are not limited thereto.

The phosphorus source may include monopotassium phosphate, dipotassium phosphate, or corresponding sodium-containing salts, *etc.* Examples of the inorganic compound may include sodium chloride, calcium chloride, iron chloride, magnesium sulfate, iron sulfate, manganese sulfate, calcium carbonate, *etc.*

Additionally, the medium may include vitamins and/or appropriate precursors, *etc.* These media or precursors may be added to a culture medium in a batch culture or continuous manner, but are not limited thereto.

In the present disclosure, the pH of a culture medium may be adjusted during the culture of the microorganism by adding a compound such as ammonium hydroxide, potassium hydroxide, ammonia, phosphoric acid, sulfuric acid, *etc.* to the culture medium in an appropriate manner. Additionally, during the culture, an antifoaming agent such as a fatty acid polyglycol ester may be added to prevent foam generation. In addition, oxygen or oxygen-containing gas may be injected into the culture medium in order to maintain an aerobic state of the culture medium; or no gas may be injected or nitrogen, hydrogen, or carbon dioxide gas may be injected into the culture medium in order to maintain an anaerobic or microaerobic state.

The temperature of the culture medium may be in a range of 25°C to 40°C, and more specifically 28°C to 37°C, but is not limited thereto. The culture may be continued until the useful materials are obtained in desired amounts, and specifically for 1 hour to 100 hours, but is not limited thereto.

The method of producing L-histidine may include a step of recovering L-histidine from one or more materials selected from the microorganism, the medium, the culture medium thereof, the supernatant of the culture medium, the extract of the culture medium, and the lysate of the microorganism, after the culturing step.

In the step of recovering, L-histidine, which is the target material, may be recovered from the culture solution using a suitable method known in the art according to the method of culturing the microorganism of the present disclosure, for example, a batch, continuous, or fed-batch culture method. For example, to recover L-histidine, methods, such as precipitation, centrifugation, filtration, chromatography, crystallization, *etc.,* may be used. For example, a supernatant obtained by removing a biomass by centrifuging the culture medium at a low speed may be separated through ion-exchange chromatography, but is not limited thereto.

The step of recovering may include a purification process.

Hereinafter, the present disclosure will be described in more detail with reference to Examples and Experimental Examples. However, these Examples and Experimental Examples are for illustrative purposes only, and the scope of the present disclosure is not intended to be limited by these Examples and Experimental Examples.

### Example 1. Preparation of Artificial Mutant Strain Having High Histidine Producing Ability

To obtain an artificial mutant strain having high L-histidine producing ability, a mutation in a microorganism was induced by way of the following method.

In detail, KCCM11795P (Korean Patent Application No. 10-2016-0030092), a histidine-producing strain prepared from *Corynebacterium glutamicum* ATCC13032 by treatment with NTG, was used to obtain a mutant strain. KCCM11795P strain activated by culturing in an activation medium for 16 hours was seeded in a seed medium and cultured for 14 hours. 5 mL of the culture medium was recovered. The recovered culture medium was washed with 100 mM citric buffer, and then *N*-methyl-*N'*-nitro-*N-*nitrosoguanidine (NTG) was added to a concentration of 200 mg/L and treated for 20 minutes, followed by washing with 100 mM phosphate buffer. A mortality rate was calculated by smearing the NTG-treated strain on a minimal medium, and as a result, the mortality rate was 85%.

To obtain a variant having resistance to 1,2,4-triazole-3-alanine (TRA), which is a derivative of L-histidine, the NTG-treated strain was smeared on a minimal medium to which 1,2,4-triazole-3-alanine was added at a concentration of 0.2 g/L, 0.5 g/L, or 1 g/L, and cultured at 30°C for 5 days. Thus, among the variants found at the three concentrations, an artificial mutant strain having 1,2,4-triazole-3-alanine resistance and the highest histidine producing ability was obtained, and this strain was named as CA14-0682.

### <Activation medium>

1% beef extract, 1% polypeptone, 0.5% sodium chloride, 1% yeast extract, 2% agar, pH 7.2

### <Seed medium>

5% glucose, 1% bactopeptone, 0.25% sodium chloride, 1% yeast extract, 0.4% urea, pH 7.2

### <Minimal medium>

1.0% glucose, 0.4% ammonium sulfate, 0.04% magnesium sulfate, 0.1% potassium dihydrogen phosphate, 0.1% urea, 0.001% thiamine, 200 µg/L biotin, 2% agar, pH 7.0

To examine the L-histidine producing ability and L-glycine production amount of the selected CA14-0682 strain, the strain was cultured by way of the following method. Each strain was seeded into a 250 mL corner-baffle flask containing 25 mL of the seed medium and cultured at 30°C for 20 hours with shaking at 200 rpm. Then, 1 mL of the seed culture solution was seeded into a 250 mL corner-baffle flask containing 25 mL of a production medium and cultured at 30°C for 24 hours with shaking at 200 rpm. After completion of the culture, the production amounts of L-histidine and L-glycine were measured via HPLC.

### <Production medium>

5% glucose, 2% ammonium sulfate, 0.1% potassium dihydrogen phosphate, 0.05% magnesium sulfate heptahydrate, 2.0% corn steep liquor (CSL), 200 µg/L biotin, calcium carbonate, pH 7.2.

**[Table 1]**

| Production amounts of L-histidine and L-glycine of CA14-0682 strain | | | | |
|---|---|---|---|---|
| | OD | Consumed glucose (g/L) | Production amount of histidine (g/L) | Production amount of glycine (g/L) |
| KCCM11795P | 110.2 | 100 | 2.99 | 1.41 |
| CA14-0682 | 50.1 | 100 | 14.25 | 6.99 |

The culture results showed that the artificial mutant strain CA14-0682 having resistance to high concentration of TRA had the L-histidine production ability of a yield of about 15%.

The CA14-0682 strain was deposited at the Korean Culture Center of Microorganisms (KCCM) and assigned Accession No. KCCM 80179.

### Example 2. Preparation of Vector for Introducing Corynebacterium ammoniagenes-Derived Glycine Transporter (CycA(Cam))

In order to insert a gene cycA (hereinafter referred to as cycA(cam), SEQ ID NO: 2) encoding a *Corynebacterium ammoniagenes*-derived CycA protein (hereinafter referred to as CycA(Cam), SEQ ID NO: 1) into the chromosome of *Corynebacterium glutamicum,* purU in *Corynebacterium glutamicum* was used as an insertion site *(*Journal of Biotechnology 104, 5-25 Jorn Kalinowski et al., 2003). In order to prepare vectors for purU deletion and target gene insertion, PCR was performed using the chromosome of ATCC13032 as a template and primer pairs of SEQ ID NOS: 3 and 4 and SEQ ID NOS: 5 and 6, respectively. PfuUltra^{™} high-fidelity DNA polymerase (Stratagene) was used as a polymerase for the PCR reaction. PCR conditions were as follows: 28 cycles of denaturation at 95°C for 30 seconds; annealing at 55°C for 30 seconds; and polymerization at 72°C for 2 minutes, followed by polymerization at 72°C for 5 minutes. As a result, DNA fragments of 1606 bp for del-purU (SEQ ID NO: 7) and 1625 bp for del-purU (SEQ ID NO: 8) were each obtained. The DNA products thus obtained were purified using a PCR purification kit (QIAGEN) and cloned into a pDZ (Korean Patent No. 10-0924065) vector using an Infusion Cloning Kit (TaKaRa) to prepare pDZΔpurU, which is a vector for purU deletion and target gene insertion.

In order to obtain a cycA(Cam) DNA fragment linked with a promoter (hereinafter referred to as Pn-cycA(Cam)), PCR was performed using the chromosome of *Corynebacterium ammoniagenes* ATCC6872 as a template and a primer pair of SEQ ID NOS: 9 and 10. PfuUltra^{™} high-fidelity DNA polymerase (Stratagene) was used as a polymerase for the PCR reaction. PCR conditions were as follows: 28 cycles of denaturation at 95°C for 30 seconds; annealing at 55°C for 30 seconds; and polymerization at 72°C for 90 seconds, followed by polymerization at 72°C for 5 minutes. As a result, a 1970 bp Pn-cycA(cam) DNA fragment was obtained. This amplified product was purified using a PCR purification kit (QIAGEN) and used as an insert DNA fragment for the preparation of a vector (SEQ ID NO: 11). The obtained DNA product was purified using a PCR purification kit (QIAGEN) and cloned into the prepared pDZΔpurU vector using an Infusion Cloning Kit (TaKaRa) to prepare pDZΔpurU::Pn-cycA(Cam), which is a cycA(Cam)-introduced vector.

### Example 3. Preparation of Strain Introduced with CA14-0682 Strain-Derived Glycine Transporter and Evaluation of Histidine Producing Ability

The vector pDZΔpurU::Pn-cycA(cam) prepared in Example 2 was transformed into CA14-0682 strain and subjected to secondary crossover to thereby prepare a strain in which purU gene on the chromosome was substituted with Pn-cycA(cam). This strain was named as CA14-0682ΔpurU::Pn-cycA(cam).

To examine L-histidine producing ability and L-glycine production amount of the prepared CA14-0682ΔpurU strain and CA14-0682ΔpurU::Pn-cycA(Cam) strain, these were cultured in the same manner as in Example 1.

**[Table 2]**

| Production amounts of L-histidine and L-glycine of strain introduced with CA14-0682-derived cycA(cam) | | | | |
|---|---|---|---|---|
| | OD | Consumed glucose (g/L) | Production amount of histidine (g/L) | Production amount of glycine (g/L) |
| CA14-0682 | 50.2 | 100 | 14.85 | 7.41 |
| CA14-0682ΔpurU | 50.1 | 100 | 14.88 | 7.42 |
| CA14-0682ΔpurU: :PncycA(Cam) | 49.7 | 100 | 15.49 | 6.51 |

As a result of the evaluation, the parent strain CA14-0682 showed L-histidine production of 14.85 g/L and L-glycine production of 7.41 g/L, and the purU-deleted strain showed an L-histidine producing ability equivalent to that of the parent strain. In contrast, CA14-0682ΔpurU: :Pn-cycA(Cam) strain showed a 4.3% increase in L-histidine production and a 13.8% decrease in L-glycine production. Therefore, it was confirmed that when extracellular L-glycine is imported into cells through introduction of the glycine transport gene, the L-histidine producing ability may be increased.

### Example 4. Preparation of CycA(Cam)-Overexpressing Recombinant Vector

In order to more strongly express intracellular cycA(Cam), a cycA(Cam)-overexpressing recombinant vector was prepared. pcj7 (Korean Patent No. 10-0620092), which is a known promoter derived from a microorganism of the *Corynebacterium,* and a known promoter (hereinafter referred to as PglyA) of glyA, which is a gene encoding serine hydroxymethyltransferase, were used.

In order to obtain a DNA fragment of the pcj7 promoter, PCR was performed using p117-cj7-gfp including pcj7 as a template. PfuUltra^{™} high-fidelity DNA polymerase (Stratagene) was used as a polymerase for the PCR reaction. The PCR reaction was performed using primers of SEQ ID NO: 12 and SEQ ID NO: 13 under the following conditions: 28 cycles of denaturation at 95°C for 30 seconds; annealing at 55°C for 30 seconds; and polymerization at 72°C for 30 seconds, followed by polymerization at 72°C for 1 minute. A PCR product thus amplified was purified using a PCR purification kit (QIAGEN) to obtain a pcj7 fragment of 350 bp.

In order to obtain a cycA(Cam) DNA fragment including a part of the pcj7 sequence in 5', PCR was performed using the chromosome of *Corynebacterium ammoniagenes* ATCC6872 as a template. PfuUltra^{™} high-fidelity DNA polymerase (Stratagene) was used as a polymerase for the PCR reaction. The PCR reaction was performed using primers of SEQ ID NO: 14 and SEQ ID NO: 10 under the following conditions: 28 cycles of denaturation at 95°C for 30 seconds; annealing at 55°C for 30 seconds; and polymerization at 72°C for 30 seconds, followed by polymerization at 72°C for 1 minute. A PCR product thus amplified was purified using a PCR purification kit (QIAGEN) to obtain a cycA(Cam) fragment of 1647 bp including a part of the pcj7 sequence in 5'.

Sewing PCR was performed using the pcj7 fragment and the cycA(Cam) fragment obtained as above as templates and primers of SEQ ID NO: 12 and SEQ ID NO: 10. The PCR reaction was performed under the following conditions: 28 cycles of denaturation at 95°C for 30 seconds; annealing at 55°C for 30 seconds; and polymerization at 72°C for 2 minutes, followed by polymerization at 72°C for 5 minutes. As a result, a pcj7-cycA(Cam) gene fragment of 1964 bp was obtained, and this amplification product was purified using a PCR Purification kit (QIAGEN) and used as an insert DNA fragment for vector preparation (SEQ ID NO: 15). The obtained DNA product was purified using a PCR Purification kit (QIAGEN), and then cloned into the prepared pDZΔpurU vector using an Infusion Cloning Kit (TaKaRa) to prepare pDZΔpurU::pcj7-cycA(Cam), which is a vector for replacing the purU gene with the pcj7-cycA(Cam) gene.

Further, in order to obtain a PglyA DNA fragment, PCR was performed using the chromosome of ATCC13032 as a template. PfuUltra^{™} high-fidelity DNA polymerase (Stratagene) was used as a polymerase for the PCR reaction. The PCR reaction was performed using primers of SEQ ID NO: 16 and SEQ ID NO: 17 under the following conditions: 28 cycles of denaturation at 95°C for 30 seconds; annealing at 55°C for 30 seconds; and polymerization at 72°C for 30 seconds, followed by polymerization at 72°C for 1 minute. A PCR product thus amplified was purified using a PCR purification kit (QIAGEN) to obtain a PglyA fragment of 340 bp.

In order to obtain a cycA(Cam) DNA fragment including a part of the PglyA sequence in 5', PCR was performed using the chromosome of *Corynebacterium ammoniagenes* ATCC6872 as a template. PfuUltra^{™} high-fidelity DNA polymerase (Stratagene) was used as a polymerase for the PCR reaction. The PCR reaction was performed using primers of SEQ ID NO: 18 and SEQ ID NO: 10 under the following conditions: 28 cycles of denaturation at 95°C for 30 seconds; annealing at 55°C for 30 seconds; and polymerization at 72°C for 30 seconds, followed by polymerization at 72°C for 1 minute. A PCR product thus amplified was purified using a PCR purification kit (QIAGEN) to obtain a cycA(Cam) fragment of 1647 bp including a part of the PglyA sequence in 5'.

Sewing PCR was performed using the PglyA fragment and the cycA(Cam) fragment obtained as above as templates and primers of SEQ ID NO: 16 and SEQ ID NO: 10. The PCR reaction was performed under the following conditions: 28 cycles of denaturation at 95°C for 30 seconds; annealing at 55°C for 30 seconds; and polymerization at 72°C for 2 minutes, followed by polymerization at 72°C for 5 minutes. As a result, a PglyA-cycA(Cam) gene fragment of 1963 bp was obtained, and this amplification product was purified using a PCR Purification kit (QIAGEN) and used as an insert DNA fragment for vector preparation (SEQ ID NO: 19). The obtained DNA product was purified using a PCR Purification kit (QIAGEN), and then cloned into the prepared pDZΔpurU vector using an Infusion Cloning Kit (TaKaRa) to prepare pDZΔpurU::PglyA-cycA(Cam), which is a vector for replacing the purU gene with the PglyA-cycA(Cam) gene.

### Example 5. Preparation of Vector for Introducing E. coli-Derived Glycine Transporter (CycA(Eco))

Meanwhile, to compare *Corynebacterium ammoniagenes*-derived CycA protein and activity thereof, a vector was prepared for introducing pcj7 operably linked with cycA (hereinafter referred to as cycA(Eco), SEQ ID NO: 21), which is a gene encoding *E. coli* K-12-derived CycA protein previously disclosed (hereinafter referred to as CycA(Eco), SEQ ID NO: 20) *(*Microbiology, 141(Pt 1); 133-40, 1995).

To obtain a DNA fragment of pcj7 promoter, PCR was performed using p117-cj7-gfp including pcj7 as a template. PfuUltra^{™} high-fidelity DNA polymerase (Stratagene) was used as a polymerase for the PCR reaction. The PCR reaction was performed using primers of SEQ ID NO: 12 and SEQ ID NO: 22 under the following conditions: 28 cycles of denaturation at 95°C for 30 seconds; annealing at 55°C for 30 seconds; and polymerization at 72°C for 30 seconds, followed by polymerization at 72°C for 1 minute. A PCR product thus amplified was purified using a PCR purification kit (QIAGEN) to obtain a pcj7 fragment of 350 bp.

In order to obtain a cycA(Eco) DNA fragment including a part of the pcj7 sequence in 5', PCR was performed using the chromosome *of E. coli* K-12 W3110 as a template and primers of SEQ ID NO: 23 and SEQ ID NO: 24. PfuUltra^{™} high-fidelity DNA polymerase (Stratagene) was used as a polymerase for the PCR reaction. The PCR conditions were as follows: 28 cycles of denaturation at 95°C for 30 seconds; annealing at 55°C for 30 seconds; and polymerization at 72°C for 1 minute, followed by polymerization at 72°C for 5 minutes. As a result, a cycA(Eco) gene fragment of 1659 bp was obtained, and this amplification product was purified using a PCR purification kit (QIAGEN) and used as an insert DNA fragment for vector preparation.

Sewing PCR was performed using the pcj7 fragment and the cycA(Eco) fragment as templates and primers of SEQ ID NO: 12 and SEQ ID NO: 24. The PCR reaction was performed under the following conditions: 28 cycles of denaturation at 95°C for 30 seconds; annealing at 55°C for 30 seconds; and polymerization at 72°C for 90 seconds, followed by polymerization at 72°C for 5 minutes. As a result, a pcj7-cycA(Eco) gene fragment of 1985 bp was obtained (SEQ ID NO: 25). This amplification product was purified using a PCR Purification kit (QIAGEN), and then cloned into the pDZΔpurU vector using an Infusion Cloning Kit (TaKaRa) in accordance with the provided manual to prepare pDZΔpurU::pcj7-cycA(Eco), which is a vector for replacing the purU gene with the pcj7-cycA(Eco) gene.

### Example 6. Preparation of CA14-0682-Derived cycA(Cam) or cycA(Eco)-Overexpressing Strain and Comparison of Histidine Producing Ability

In order to prepare a cycA(Cam) or cycA(Eco)-overexpressing strain using a CA14-0682 strain as a parent strain, the prepared four vectors (pDZΔpurU, pDZΔpurU::pcj7-cycA(Cam), pDZΔpurU::PglyA-cycA(Cam), and pDZΔpurU::pcj7-cycA(Eco)) were each transformed into the CA14-0682 strain via electroporation. Secondary crossover was performed to obtain a strain in which deletion of purU gene on the chromosome and substitution in the form of pcj7-cycA(Cam) or PglyA-cycA(Cam) or pcj7-cycA(Eco) were performed, respectively. Through this process, four strains (CA14-0682ΔpurU, CA14-0682ΔpurU: :pcj7-cycA(Cam), CA14-0682ΔpurU: :PglyAcycA(Cam), and CA14-0682ΔpurU::pcj7-cycA(Eco)) were prepared.

In order to examine L-histidine producing ability and L-glycine production amount of the prepared four strains, the strains were each cultured in the same manner as in Example 1.

**[Table 3]**

| Production amounts of L-histidine and L-glycine of strain introduced with CA14-0682-derived cycA | | | | |
|---|---|---|---|---|
| | OD | Consumed glucose (g/L) | Production amount of histidine (g/L) | Production amount of glycine (g/L) |
| CA14-0682 | 50.3 | 100 | 15.11 | 7.46 |
| CA14-0682ΔpurU | 50.5 | 100 | 15.05 | 7.42 |
| CA14-0682ΔpurU::pcj7_cycA(Cam) | 40.1 | 100 | 16.18 | 5.99 |
| CA14-0682ΔpurU::Pgly A_cycA(Cam) | 44.7 | 100 | 16.14 | 5.89 |
| CA14-0682ΔpurU::pcj7_cycA(Eco) | 51.3 | 100 | 15.01 | 7.25 |

As a result of the evaluation, the CA14-0682ΔpurU::pcj7-cycA(Eco) strain introduced with E. coli-derived cycA showed rare uptake of Gly and L-histidine producing ability equivalent to or lower than that of the parent strain. In contrast, the CA14-0682ΔpurU::pcj7-cycA(Cam) strain and the CA14-0682ΔpurU::PglyA_cycA(Cam) strain, each enhanced by introduction with *Corynebacterium ammoniagenes*-derived cycA, showed a decrease in the Gly producing ability and 7.1% and 6.8% increase in histidine producing ability as compared with the parent strain, respectively. Therefore, it was confirmed that *Corynebacterium ammoniagenes-derived* cycA introduced into *Corynebacterium glutamicum* showed the high glycine transport ability to exhibit a high effect on the histidine producing ability due to the imported glycine as compared with *E. coli*-derived cycA. It was also confirmed that when cycA(Cam) is introduced and expressed through the glyA promoter, it is more beneficial in obtaining a cell mass. The CA14-0682ΔpurU::PglyA-cycA(Cam) strain was named as a CA14-0682-cycA(Cam) strain.

### Example 7. Preparation of Vector for Introducing Corynebacterium ammoniagenes-Derived Glycine Cleavage System

Since it had been previously confirmed that the activity of the glycine transporter had an effect on the increase in the histidine producing ability, the histidine producing ability was examined when the intracellular utilization of glycine imported into the cells was further increased. In detail, a glycine cleavage system (hereinafter referred to as a GCV system) was introduced. With regard to the *Corynebacterium glutamicum* strain, among the six proteins constituting the GCV system, only genes encoding L-proteins, such as LipB and LipA, are known, but genes encoding the other three proteins are not known. Therefore, in order to introduce the GCV system derived from *Corynebacterium ammoniagenes,* vectors for introducing genes (gcvP (SEQ ID NO: 31), gcvT (SEQ ID NO: 32), gcvH (SEQ ID NO: 33), lipA (SEQ ID NO: 34), and lipB (SEQ ID NO: 35)) encoding P-protein (SEQ ID NO: 26), T-protein (SEQ ID NO: 27), H-protein (SEQ ID NO: 28), LipA (SEQ ID NO: 29), and LipB (SEQ ID NO: 30)) were prepared. The genes form two pairs of operons (gcvP-gcvT and gcvH-lipB-lipA) on the chromosome of *Corynebacterium ammoniagenes* (hereinafter referred to as gcvPT and gcvH-lipBA). To introduce the GCV system, NCgl2131 gene among genes encoding transposon of *Corynebacterium glutamicum* was used as an insertion site (Journal of Biotechnology 104, 5-25 Jorn Kalinowski et al., 2003). In order to replace the NCgl2131 gene with the GCV system genes, a vector for NCgl2131 deletion and target gene insertion was prepared. To prepare the vector, PCR was performed using the chromosome of ATCC13032 as a template and primer pairs of SEQ ID NOS: 36 and 37 and SEQ ID NOS: 38 and 39, respectively. PfuUltra^{™} high-fidelity DNA polymerase (Stratagene) was used as a polymerase for the PCR reaction. The PCR conditions were as follows: 28 cycles of denaturation at 95°C for 30 seconds; annealing at 55°C for 30 seconds; and polymerization at 72°C for 2 minutes, followed by polymerization at 72°C for 5 minutes. As a result, DNA fragments of 531 bp for del-N2131L (SEQ ID NO: 40) and 555 bp for del-N2131R (SEQ ID NO: 41) were each obtained. The obtained DNA product was purified using a PCR purification kit (QIAGEN) and then cloned into the pDZ vector using an Infusion Cloning Kit (TaKaRa) to prepare pDZΔN2131, which is a vector for NCgl2131 gene deletion and target gene insertion.

In order to obtain a gcvPT gene fragment linked with a promoter (hereinafter referred to as Pn_gcvPT(cam)), PCR was performed using the chromosome of *Corynebacterium ammoniagenes* ATCC6872 as a template and primers of SEQ ID NO: 42 and SEQ ID NO: 43. PfuUltra^{™} high-fidelity DNA polymerase (Stratagene) was used as a polymerase for the PCR reaction. The PCR conditions were as follows: 28 cycles of denaturation at 95°C for 30 seconds; annealing at 55°C for 30 seconds; and polymerization at 72°C for 5 minutes, followed by polymerization at 72°C for 7 minutes. As a result, a Pn_gcvPT(Cam) gene fragment of 4499 bp including the promoter was obtained. This amplification product was purified using a PCR purification kit (QIAGEN) and used as an insert DNA fragment for vector preparation (SEQ ID NO: 44).

In order to obtain a gcvH-lipBA gene fragment linked with a promoter (hereinafter referred to as Pn_gcvH-lipBA(Cam)), PCR was performed using the chromosome of *Corynebacterium ammoniagenes* ATCC6872 as a template and primers of SEQ ID NO: 45 and SEQ ID NO: 46. PfuUltra^{™} high-fidelity DNA polymerase (Stratagene) was used as a polymerase for the PCR reaction. The PCR conditions were as follows: 28 cycles of denaturation at 95°C for 30 seconds; annealing at 55°C for 30 seconds; and polymerization at 72°C for 1 minute, followed by polymerization at 72°C for 7 minutes. As a result, a Pn_gcvH-lipBA(Cam) gene fragment of 3053 bp including the promoter was obtained. This amplification product was purified using a PCR purification kit (QIAGEN) and used as an insert DNA fragment for vector preparation (SEQ ID NO: 47).

Sewing PCR was performed using the Pn_gcvPT(Cam) fragment and the Pn_gcvH-lipBA(Cam) fragment obtained as above as templates and primers of SEQ ID NO: 42 and SEQ ID NO: 46. The PCR reaction was performed under the following conditions: 28 cycles of denaturation at 95°C for 30 seconds; annealing at 55°C for 30 seconds; and polymerization at 72°C for 10 minutes, followed by polymerization at 72°C for 12 minutes. As a result, a Pn_gcvPT(Cam)_Pn-gcvH-lipBA(Cam) gene fragment of 8259 bp was obtained, and this amplification product was purified using a PCR Purification kit (QIAGEN) and cloned into the prepared pDZΔN2131 vector using an Infusion Cloning Kit (TaKaRa) to prepare pDZΔN2131::GCV(Cam), which is a vector for replacing the NCgl2131 gene with the Pn_gcvPT(Cam)-Pn_gcvH-lipBA(Cam) gene.

### Example 8. Preparation of CA14-0682-Derived Strain Introduced with Glycine Cleavage System and Glycine Transporter and Evaluation of Histidine Producing Ability

The pDZΔN2131 and pDZΔN2131::GCV(Cam) vectors prepared in Example 7 were transformed into CA14-0682 strain and CA14-0682-cycA(Cam) strain, respectively. Then, secondary crossover was performed to prepare a strain (CA14-0682-cycA(Cam)ΔN2131), in which the NCgl2131 gene was deleted, a strain into which the glycine cleavage system alone was introduced, and two strains (CA14-0682ΔN2131::GCV(Cam) and CA14-0682-cycA(Cam)ΔN2131::GCV(Cam)) into which both of the glycine cleavage system and the glycine transporter were introduced. In order to examine L-histidine producing ability and L-glycine production amount of the prepared CA14-0682-cycA(Cam)ΔN2131 strain, CA14-0682ΔN2131::GCV(Cam) strain, and CA14-0682-cycA(Cam)ΔN2131: :GCV(Cam) strain, these were cultured in the same manner as in Example 1, respectively.

**[Table 4]**

| Production amounts of L-histidine and L-glycine of strain introduced with CA14-0682-derived cycA(cam) and glycine cleavage system | | | | |
|---|---|---|---|---|
| | OD | Consumed glucose (g/L) | Production amount of histidine (g/L) | Production amount of glycine (g/L) |
| CA14-0682 | 53.6 | 100 | 15.05 | 7.47 |
| CA14-0682-cycA(Cam)ΔN2131 | 45.1 | 100 | 16.19 | 6.68 |
| CA14-0682ΔN2131::GCV(Cam) | 48.9 | 100 | 16.52 | 4.72 |
| CA14-0682-cycA(Cam)ΔN2131::GCV(Cam) | 42.3 | 100 | 17.11 | 2.31 |

As a result of the evaluation, the CA14-0682-cycA(Cam)ΔN2131 strain, into which only the glycine transporter cycA(Cam) was introduced, showed a 7.6% increase in the histidine production amount and a 10.6% decrease in the glycine production amount as compared with the CA14-0682 strain, indicating that these results are equivalent to those of the CA14-0682ΔpurU::PglyA_cycA(Cam) (named as CA14-0682-cycA(Cam)) strain of Table 3. The CA14-0682ΔN2131::GCV(Cam) strain, into which the glycine cleavage system was introduced, showed a 9.8% increase in the histidine production amount and a 36.8% decrease in the glycine production amount as compared with the CA14-0682 strain. In contrast, the CA14-0682-cycA(Cam)ΔN2131::GCV(Cam) strain, prepared by additionally introducing GCV into the CA14-0682-cycA(Cam)ΔN2131 strain, showed a 13.7% increase in the histidine production amount and a 69.1% decrease in the glycine production amount as compared with the parent strain. Therefore, it was confirmed that although introduction of only the glycine transporter gene or glycine cleavage gene exhibits the effects of increasing histidine productivity and decreasing glycine productivity, introduction of both further increases histidine productivity with degradation of glycine produced in cells.

### Example 9. Preparation of Wild-type Corynebacterium glutamicum-Derived Strain for Producing L-Histidine

Next, in order to examine the effects of introducing CycA and the GCV system into the wild-type *Corynebacterium glutamicum* strain, an L-histidine-producing strain was developed from the wild-type *Corynebacterium glutamicum* ATCC13032 strain.

### Example 9-1: Introduction of HisG Polypeptide Mutation

First, in order to release feedback inhibition of HisG polypeptide, which is a first enzyme in the L-histidine biosynthesis pathway, amino acids at positions 233 and 235 from the N-terminus of HisG were substituted from glycine to histidine (hereinafter referred to as G233H mutant) and from threonine to glutamine (hereinafter referred to as T235Q) (SEQ ID NO: 48) at the same time *(*ACS Synth. Biol., 2014, 3(1), pp. 21-29).

In detail, in order to prepare a vector for inserting a hisG polypeptide mutation, gene fragments of the upstream region (hereinafter referred to as G233H,T235Q-5') of residues at positions 233 and 235 of the hisG polypeptide and the downstream region (hereinafter referred to as G233H,T235Q-3') of residues at positions 233 and 235 of the hisG polypeptide were obtained by PCR using the chromosomal DNA of *Corynebacterium glutamicum* ATCC13032 as a template and primers of SEQ ID NOS: 49 and 50 and SEQ ID NOS: 51 and 52, respectively. Solg^{™} Pfu-X DNA polymerase was used as a polymerase. The PCR amplification conditions were as follows: denaturation at 95°C for 5 minutes, 30 cycles of denaturation at 95°C for 30 seconds; annealing at 60°C for 30 seconds; and polymerization at 72°C for 30 seconds, followed by polymerization at 72°C for 5 minutes.

The amplified G233H,T235Q-5' fragment and G233H,T235Q-3' fragment were cloned into pDZ using a Gibson assembly method (DG Gibson et al., NATURE METHODS, VOL. 6 NO. 5, MAY 2009, NEBuilder HiFi DNA Assembly Master Mix) to prepare pDZ-hisG (G233H, T235Q), which is a vector introduced with hisG polypeptide mutation.

The prepared pDZ-hisG (G233H, T235Q) vector was transformed into a wild-type *Corynebacterium glutamicum* ATCC13032 strain by electroporation, and then secondary crossover was performed to obtain a strain in which amino acids at positions 233 and 235 of the HisG polypeptide were substituted from glycine to histidine and from threonine to glutamine on the chromosome. The corresponding genetic manipulation was identified by PCR using SEQ ID NO: 53 and SEQ ID NO: 54, which are able to amplify the outer region of the gene-inserted homologous recombination upstream and downstream regions, and by sequencing, and the strain was named as CA14-0011.

### Example 9-2: Enhancement of Histidine Biosynthesis Pathway

Next, to enhance the L-histidine biosynthesis pathway, biosynthetic genes separated into a total of four operons were prepared in the form of a promoter-substituted cluster, which was then introduced. In detail, biosynthetic genes separated into a total of four operons (hisE-hisG, hisA-impA-hisF-hisI, hisD-hisC-hisB, and cg0911-hisN) were operably linked to three known synthetic promoters (lysCP1 (Korean Patent No. 10-0930203), pcj7, or SPL13 (Korean Patent No. 10-1783170 B1)) or to gapA gene promoter, and respective operons were clustered and then introduced at once. Ncgl1108 gene encoding *gamma*-aminobutyrate permease was used as an insertion site *(*Microb Biotechnol. 2014 Jan;7(1):5-25).

A specific experimental method is as follows. In order to prepare a vector for NCgl1108 gene deletion, gene fragments of the upstream region of NCgl1108 (hereinafter referred to as N1108-5') and the downstream region of Ncgl1108 (hereinafter referred to as N1108-3') were obtained by PCR using the chromosomal DNA of *Corynebacterium glutamicum* ATCC13032 as a template and primers of SEQ ID NOS: 55 and 56 and SEQ ID NOS: 57 and 58, respectively. Solg^{™} Pfu-X DNA polymerase was used as a polymerase. The PCR amplification conditions were as follows: denaturation at 95°C for 5 minutes, 30 cycles of denaturation at 95°C for 30 seconds; annealing at 60°C for 30 seconds; and polymerization at 72°C for 60 seconds, followed by polymerization at 72°C for 5 minutes. The amplified N1108-5' fragment and N1108-3' fragment were cloned into pDZ using a Gibson assembly method to prepare a pDZΔN1108 vector, which is an NCgl1108-deleted vector.

The prepared pDZ-ΔNCgl1108 vector was transformed into a CA14-0011 strain by electroporation, and then secondary crossover was performed to obtain a strain in which the NCgl1108 gene on the chromosome was broken. The corresponding genetic manipulation was identified by PCR using SEQ ID NO: 59 and SEQ ID NO: 60, which are able to amplify the outer region of the gene-broken homologous recombination upstream and downstream regions, and by sequencing, and the strain was named as CA14-0736.

To enhance a histidine biosynthetic cluster, four operon gene clusters and promoter regions to be substituted were obtained. A lysCP1 promoter fragment and a hisE-hisG fragment, a gapA promoter fragment and a hisA-impA-hisF-hisI fragment, a SPL13 fragment and a hisD-hisC-hisB fragment, and a pcj7 fragment and a cg0911-hisN fragment were obtained.

To obtain the lysCP1 DNA fragment, PCR was performed using the chromosome of KCCM10919P strain (Korean Patent No. 10-0930203) as a template. PfuUltra^{™} high-fidelity DNA polymerase (Stratagene) was used as a polymerase for the PCR reaction. The PCR reaction was performed using primers of SEQ ID NO: 61 and SEQ ID NO: 62 under the following conditions: 28 cycles of denaturation at 95°C for 30 seconds; annealing at 55°C for 30 seconds; and polymerization at 72°C for 30 seconds, followed by polymerization at 72°C for 1 minute. The PCR product thus amplified was purified using a PCR Purification kit (QIAGEN) to obtain the lysCP1 fragment.

To obtain the hisE-hisG gene fragment, PCR was performed using the chromosome of CA14-0011 strain as a template. The PCR reaction was performed using primers of SEQ ID NO: 63 and SEQ ID NO: 64 under the following conditions: 28 cycles of denaturation at 95°C for 30 seconds; annealing at 55°C for 30 seconds; and polymerization at 72°C for 2 minutes, followed by polymerization at 72°C for 5 minutes. The PCR product thus amplified was purified using a PCR Purification kit (QIAGEN) to obtain the hisE-hisG fragment.

To obtain a promoter DNA fragment of *Corynebacterium glutamicum*-derived gapA gene (hereinafter referred to as PgapA), PCR was performed using the chromosome of *Corynebacterium glutamicum* ATCC13032 as a template. The PCR reaction was performed using primers of SEQ ID NO: 65 and SEQ ID NO: 66 under the following conditions: 28 cycles of denaturation at 95°C for 30 seconds; annealing at 55°C for 30 seconds; and polymerization at 72°C for 2 minutes, followed by polymerization at 72°C for 5 minutes. The PCR product thus amplified was purified using a PCR Purification kit (QIAGEN) to obtain the PgapA fragment.

To obtain a hisA-impA-hisF-hisI gene fragment, PCR was performed using the chromosome of CA14-0011 strain as a template. The PCR reaction was performed using primers of SEQ ID NO: 67 and SEQ ID NO: 68 under the following conditions: 28 cycles of denaturation at 95°C for 30 seconds; annealing at 55°C for 30 seconds; and polymerization at 72°C for 2 minutes, followed by polymerization at 72°C for 5 minutes. The PCR product thus amplified was purified using a PCR Purification kit (QIAGEN) to obtain the hisA-impA-hisF-hisI fragment.

To obtain a SPL13 DNA fragment, PCR was performed using SPL13 DNA as a template. The PCR reaction was performed using primers of SEQ ID NO: 69 and SEQ ID NO: 70 under the following conditions: 28 cycles of denaturation at 95°C for 30 seconds; annealing at 55°C for 30 seconds; and polymerization at 72°C for 1 minute, followed by polymerization at 72°C for 5 minutes. The PCR product thus amplified was purified using a PCR Purification kit (QIAGEN) to obtain the SPL13 DNA fragment.

To obtain a pcj7 promoter DNA fragment, PCR was performed using p117-cj7-gfp including pcj7 as a template. PfuUltra^{™} high-fidelity DNA polymerase (Stratagene) was used as a polymerase for the PCR reaction. The PCR reaction was performed using primers of SEQ ID NO: 71 and SEQ ID NO: 72 under the following conditions: 28 cycles of denaturation at 95°C for 30 seconds; annealing at 55°C for 30 seconds; and polymerization at 72°C for 30 seconds, followed by polymerization at 72°C for 1 minute. The PCR product thus amplified was purified using a PCR Purification kit (QIAGEN) to obtain the pcj7 fragment.

To obtain a hisD-hisC-hisB gene fragment, PCR was performed using the chromosome of CA14-0011 strain as a template. The PCR reaction was performed using primers of SEQ ID NO: 73 and SEQ ID NO: 74 under the following conditions: 28 cycles of denaturation at 95°C for 30 seconds; annealing at 55°C for 30 seconds; and polymerization at 72°C for 5 minutes, followed by polymerization at 72°C for 5 minutes. The PCR product thus amplified was purified using a PCR Purification kit (QIAGEN) to obtain the hisD-hisC-hisB gene fragment.

To obtain a cg0911-hisN gene fragment, PCR was performed using the chromosome of CA14-0011 strain as a template. The PCR reaction was performed using primers of SEQ ID NO: 75 and SEQ ID NO: 76 under the following conditions: 28 cycles of denaturation at 95°C for 30 seconds; annealing at 55°C for 30 seconds; and polymerization at 72°C for 5 minutes, followed by polymerization at 72°C for 5 minutes. The PCR product thus amplified was purified using a PCR Purification kit (QIAGEN) to obtain the cg0911-hisN gene fragment.

The obtained lysCP1 DNA fragment, hisE-hisG DNA fragment, PgapA DNA fragment, hisA-impA-hisF-hisI DNA fragment, SPL13 DNA fragment, hisD-hisC-hisB DNA fragment, pcj7 DNA fragment, and cg0911-hisN DNA fragment were cloned into a pDZ-ΔNcgl1108 vector using a Gibson assembly method to prepare pDZΔNcgl1108::lysCP1_hisEG-PgapA_hisA-impA-hisFI-SPL13_HisDCB-pcj7_cg0911-hisN, which is a vector introduced with the L-histidine biosynthesis-enhanced cluster.

The prepared pDZ-ΔNcgl108::lysCP1_hisEG-PgapA_hisA-impA-hisFI-SPL13_hisDCB-pcj7_cg0911-hisN vector was transformed into a CA14-0011 strain via electroporation, and then secondary crossover was performed to obtain a strain in which biosynthetic genes were inserted into the chromosome. The corresponding genetic manipulation was identified via PCR using SEQ ID NO: 59 and SEQ ID NO: 60, which are able to amplify the outer region of the gene-inserted homologous recombination upstream and downstream regions, and via genomic sequencing, and the strain was named as CA14-0737.

The CA14-0737 strain was deposited at the Korean Culture Center of Microorganisms (KCCM), an International Depositary Authority under the Budapest Treaty, on November 27, 2018, and was assigned Accession No. KCCM12411P.

### Example 10. Preparation of Strain Introduced with CA14-0737 Strain-Derived Glycine Transporter and Glycine Cleavage System

The prepared four vectors (pDZΔpurU, pDZΔpurU::PglyA-cycA(Cam), pDZΔpurU::pcj7-cycA(Cam), and pDZΔpurU::pcj7-cycA(Eco)) were each transformed into CA14-0737 strain, and secondary crossover was performed to prepare a purU genedeleted strain, a cycA(Cam)-introduced strain, and a cycA(Eco)-introduced strain, which were named as CA14-0737ΔpurU, CA14-0737ΔpurU::PglyA-cycA(Cam), CA14-0737ΔpurU::pcj7-cycA(Cam), and CA14-0737ΔpurU::pcj7-cycA(Eco). In order to examine L-histidine producing ability and L-glycine production amount of the prepared CA14-0737ΔpurU, CA14-0737ΔpurU::PglyA-cycA(Cam), CA14-0737ΔpurU::pcj7-cycA(Cam), and CA14-0737ΔpurU::pcj7-cycA(Eco) strains, these were cultured in the same manner as in Example 1.

**[Table 5]**

| Production amounts of L-histidine and L-glycine of strain introduced with CA14-0737-derived cycA | | | | |
|---|---|---|---|---|
| | OD | Consumed glucose (g/L) | Production amount of histidine (g/L) | Production amount of glycine (g/L) |
| CA14-0737 | 88.4 | 100 | 4.11 | 2.21 |
| CA14-0737ΔpurU | 87.9 | 100 | 4.20 | 2.24 |
| CA14-0737ΔpurU: :PglyAcycA(Cam) | 87.4 | 100 | 4.93 | 1.90 |
| CA14-0737ΔpurU::pcj7-cycA(Cam) | 84.1 | 100 | 4.97 | 1.95 |
| CA14-0737ΔpurU::pcj7-cycA(Eco) | 88.9 | 100 | 4.29 | 2.20 |

As a result of the evaluation, the CA14-0737ΔpurU::pcj7-cycA(Eco) strain, into which E. coli-derived cycA was introduced, showed rare Gly uptake, and thus exhibited histidine producing ability equivalent to that of the parent strain. In contrast, the CA14-0737ΔpurU::pcj7-cycA(Cam) strain, into which the enhanced *Corynebacterium ammoniagenes*-derived cycA was introduced, showed a 20.9% increase in the histidine producing ability and a 11.8% decrease in the glycine production amount as compared with the parent strain. The strain in which cycA(Cam) was expressed through the glyA promoter also showed a 20% increase in the histidine producing ability and a 14% decrease in the glycine production amount. These results indicate that *Corynebacterium ammoniagenes*-derived cycA introduced into *Corynebacterium glutamicum* has higher glycine uptake to exhibit the higher effect of increasing histidine producing ability through the glycine than *E. coli*-derived cycA. Among these, the CA14-0737ΔpurU::PglyA-cycA(Cam) strain was named as CA14-0737-cycA(Cam).

The pDZΔN2131 and pDZΔN2131::GCV(Cam) vectors prepared in Example 7 were transformed into CA14-0737 strain and CA14-0737-cycA(Cam) strain, respectively. Then, secondary crossover was performed to prepare a strain (CA14-0737-cycA(Cam)ΔN2131), in which the NCgl2131 gene was deleted, a strain into which the glycine cleavage system alone was introduced, and two strains (CA14-0737ΔN2131::GCV(Cam), CA14-0737-cycA(Cam)ΔN2131::GCV(Cam)), into which both of the glycine cleavage system and the glycine transporter were introduced. In order to examine L-histidine producing ability and L-glycine production amount of the prepared CA14-0737-cycA(Cam)ΔN2131 strain, CA14-0737ΔN2131::GCV(Cam) strain, and CA14-0737-cycA(Cam)ΔN2131: :GCV(Cam) strain, these were cultured in the same manner as in Example 1, respectively.

**[Table 6]**

| Production amounts of L-histidine and L-glycine of strain introduced with CA14-0737-derived cycA(cam) and glycine cleavage system | | | | |
|---|---|---|---|---|
| | OD | Consumed glucose (g/L) | Production amount of histidine (g/L) | Production amount of glycine (g/L) |
| CA14-0737 | 88.1 | 100 | 4.15 | 2.17 |
| CA14-0737-cycA(Cam)ΔN2131 | 75.1 | 100 | 4.89 | 1.48 |
| CA14-0737ΔN2131::GCV(Cam) | 78.2 | 100 | 5.42 | 0.94 |
| CA14-0737-cycA(Cam)ΔN2131::GCV(Cam) | 71.3 | 100 | 5.97 | 0.46 |

As a result of the evaluation, the CA14-0737-cycA(Cam) ΔN2131 strain, into which only the glycine transporter cycA(Cam) was introduced, showed a 17.8% increase in the histidine production amount and a 13% decrease in the glycine production amount as compared with the parent strain. The CA14-0737-cycA(Cam)ΔN2131::GCV(Cam) strain, into which the glycine transporter cycA(cam) and GCV were introduced at the same time, showed a 43.9% increase in the histidine production amount and a 78.8% decrease in the glycine production amount as compared with the parent strain. The CA14-0737ΔN2131::GCV(Cam) strain, into which the glycine cleavage system was introduced, showed a 30.6% increase in the histidine production amount and a 56.7% decrease in the glycine production amount as compared with the parent strain, but glycine was still accumulated in the culture medium, and histidine productivity was also lower than that of the strain into which GCV was introduced together with cycA(Cam). Therefore, it was confirmed that although introduction of only the glycine transporter gene or glycine cleavage system exhibits the effects of increasing histidine productivity and decreasing glycine productivity, introduction of both of the glycine transporter and the glycine cleavage system further increases histidine productivity with degradation of glycine produced in cells. The CA14-0737-cycA(Cam) strain was named as CA14-0777, and the CA14-0737-cycA(Cam)ΔN2131::GCV(Cam) strain was named as CA14-0809. The two strains were deposited at the Korean Culture Center of Microorganisms (KCCM), an International Depositary Authority under the Budapest Treaty, on April 15, 2019, and was assigned Accession Nos. KCCM12488P and KCCM12489P, respectively.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

### Effect of the Invention

A microorganism for producing L-histidine of the present disclosure may have an excellent histidine producing ability, and may thereby be applied to efficient mass production of L-histidine.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA14-0737 | Accession number given by the
INTERNATIONAL DEPOSITARY AUTHORITY:
KCCM12411P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 27. 2018 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 27. 2018.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoorim Bldg., 45, Hongjenae 2ga-gil, Seodaemun-gu, Seoul, 03641, Korea Tel: 82-2-391-0950, 396-0950 Fax: 82-2-392-2859

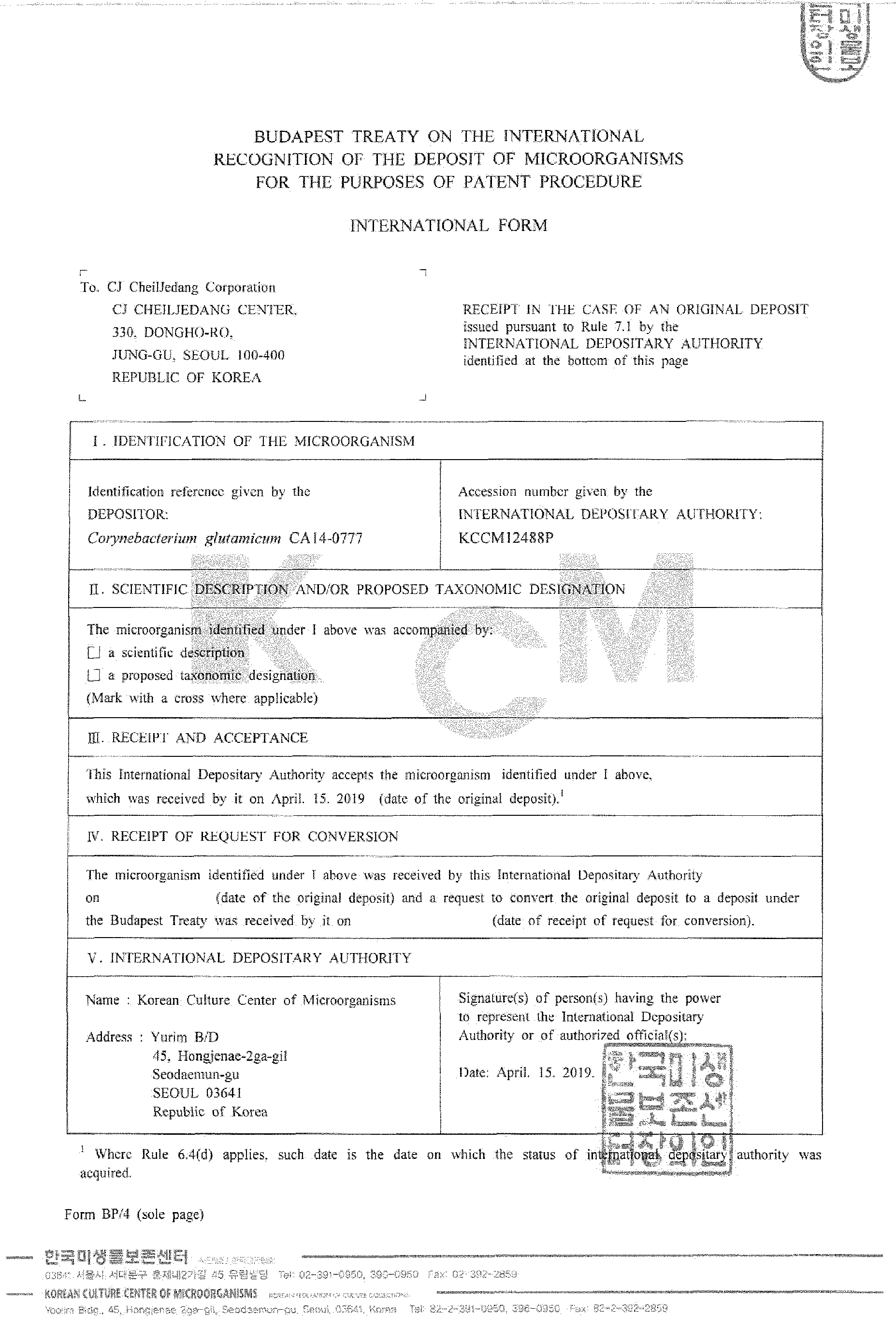
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I . IDENTIFICATION OF THE MICROORGANISM
Identification reference given by the
DEPOSITOR:
Corynebacterium glutamicum CA14-0777 | Accession number given by the
INTERNATIONAL DEPOSITARY AUTHORITY:
KCCM12488P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 15. 2019 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 15. 2019.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoorim Bldg., 45, Hongjenae 2ga-gil, Seodaemun-gu, Seoul, 03641, Korea Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA14-0809 | Accession number given by the
INTERNATIONAL DEPOSITARY AUTHORITY:
KCCM12489P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 15. 2019 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 15. 2019.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A microorganism of the genus *Corynebacterium* for producing L-histidine, the microorganism having an enhanced glycine transporter activity.

2. The microorganism of claim 1, wherein the glycine transporter is derived from *Corynebacterium ammoniagenes.*

3. The microorganism of claim 1, wherein the glycine transporter protein is a CycA protein.

4. The microorganism of claim 1, wherein the glycine transporter comprises an amino acid sequence of SEQ ID NO: 1 or an amino acid sequence having 90% or more sequence homology thereto.

5. The microorganism of claim 1, wherein activity of a glycine cleavage protein is further enhanced.

6. The microorganism of claim 1, wherein the glycine cleavage protein is one or more proteins selected from the group consisting of GcvP, GcvT, GcvH, LipB, and LipA.

7. The microorganism of claim 6, wherein the glycine cleavage protein is derived from *Corynebacterium ammoniagenes.*

8. The microorganism of claim 6, wherein the GcvP comprises an amino acid sequence of SEQ ID NO: 26, GcvT comprises an amino acid sequence of SEQ ID NO: 27, GcvH comprises an amino acid sequence of SEQ ID NO: 28, LipA comprises an amino acid sequence of SEQ ID NO: 29, and LipB comprises an amino acid sequence of SEQ ID NO: 30, or each comprises an amino acid sequence having 90% or more homology to the respective amino acid sequence.

9. The microorganism of claim 1, wherein the microorganism of the genus *Corynebacterium* for producing L-histidine is *Corynebacterium glutamicum.*

10. A composition for producing L-histidine, the composition comprising the microorganism of any one of claims 1 to 9.

11. A method of producing L-histidine, the method comprising the steps of:
- culturing the microorganism of any one of claims 1 to 9 in a medium; and
- recovering L-histidine from the microorganism and the medium.

12. Use of a microorganism of the genus *Corynebacterium* having an enhanced glycine transporter activity in the production of L-histidine.


<110>    CJ CheilJedang Corporation

<120>    Microorganisms with enhanced ability to produce L-histidine and
         methods for producing L-histidine using the same

<130>    OPA19300

<150>    KR 10-2019-0046934
<151>    2019-04-22

<160>    76

<170>    KoPatentIn 3.0

<210>    1
<211>    476
<212>    PRT
<213>    Corynebacterium ammoniagenes

<400>    1
Met Ala Ser Tyr Asp Pro Gly His Ser Gln Val Lys Asn Thr Gly Val
  1               5                  10                  15

Glu Leu Asp Ser His Val Glu Ser Asp His Leu Gln Arg Gly Leu Ser
             20                  25                  30

Asn Arg His Ile Gln Leu Ile Ala Ile Gly Gly Ala Ile Gly Thr Gly
         35                  40                  45

Leu Phe Met Gly Ser Gly Lys Thr Ile Ser Leu Ala Gly Pro Ser Val
     50                  55                  60

Ile Leu Val Tyr Gly Ile Ile Gly Phe Met Leu Phe Phe Val Met Arg
 65                  70                  75                  80

Ala Met Gly Glu Leu Leu Leu Ser Asn Leu Asn Tyr Lys Ser Leu Arg
                 85                  90                  95

Asp Ala Val Ser Asp Ile Leu Gly Pro Thr Ala Gly Phe Val Cys Gly
            100                 105                 110

Trp Thr Tyr Trp Phe Cys Trp Ile Val Thr Gly Met Ala Asp Val Val
        115                 120                 125

Ala Ile Thr Gly Tyr Thr Gln Tyr Trp Trp Pro Asp Ile Ala Leu Trp
    130                 135                 140

Ile Pro Gly Ala Leu Thr Ile Leu Leu Leu Leu Gly Leu Asn Leu Val
145                 150                 155                 160

Ala Val Arg Leu Phe Gly Glu Leu Glu Phe Trp Phe Ala Ile Ile Lys
                165                 170                 175

Leu Val Ala Ile Thr Ala Leu Ile Leu Val Gly Ala Val Met Val Ile
            180                 185                 190

Ser Arg Phe Gln Ser Pro Asp Gly Asp Ile Ala Ala Val Ser Asn Leu
        195                 200                 205

Ile Asp His Gly Gly Phe Phe Pro Asn Gly Ile Thr Gly Phe Leu Ala
    210                 215                 220

Gly Phe Gln Ile Ala Ile Phe Ala Phe Val Gly Val Glu Leu Ala Gly

225                 230                 235                 240

Thr Ala Ala Ala Glu Thr Lys Asp Pro Glu Lys Asn Leu Pro Arg Ala
                245                 250                 255

Ile Asn Ser Ile Pro Ile Arg Ile Val Val Phe Tyr Ile Leu Ala Leu
            260                 265                 270

Ala Val Ile Met Met Val Thr Pro Trp Asp Lys Val His Ser Asp Ser
        275                 280                 285

Ser Pro Phe Val Gln Met Phe Ala Leu Ala Gly Leu Pro Ala Ala Ala
    290                 295                 300

Gly Ile Ile Asn Phe Val Val Leu Thr Ser Ala Ala Ser Ser Ala Asn
305                 310                 315                 320

Ser Gly Ile Phe Ser Thr Ser Arg Met Leu Phe Gly Leu Ala Arg Glu
                325                 330                 335

Gly Gln Ala Pro Lys Arg Trp Gly Ile Leu Ser Arg Asn Gln Val Pro
            340                 345                 350

Ala Arg Gly Leu Leu Phe Ser Val Ala Cys Leu Val Pro Ser Leu Ala
        355                 360                 365

Ile Leu Tyr Ala Gly Ala Ser Val Ile Asp Ala Phe Thr Leu Ile Thr
    370                 375                 380

Thr Val Ser Ser Val Leu Phe Met Val Val Trp Ser Leu Ile Leu Ala
385                 390                 395                 400

Ala Tyr Leu Val Phe Arg Arg Lys Phe Pro Glu Arg His Ala Ala Ser
                405                 410                 415

Lys Phe Lys Val Pro Gly Gly Ala Phe Met Cys Trp Val Val Leu Ala
            420                 425                 430

Phe Phe Ala Phe Met Val Val Val Leu Thr Phe Glu Asn Asp Thr Arg
        435                 440                 445

Ser Ala Leu Met Val Thr Pro Ile Trp Phe Val Ile Leu Leu Ala Gly
    450                 455                 460

Trp Phe Ile Ser Gly Gly Val Lys Arg Ala Arg Lys
465                 470                 475

<210>   2
<211>   1431
<212>   DNA
<213>   Corynebacterium ammoniagenes

<400>   2
atggcatctt atgatcccgg gcattcacag gttaaaaata caggagtaga actcgactct      60

cacgtggagt cagaccatct ccaacgcggt cttagcaacc gacatatcca gttgattgcc     120

attggcggtg ctatcggcac cggactattt atgggatcgg gcaaaaccat ctcccttgct     180

ggtccttccg taatcttggt gtatggcatc atcggtttta tgctcttctt cgtcatgcgg     240

gcgatgggtg agcttttgct ctcgaacctc aactacaagt cattgcgtga tgccgtctcg     300

gacatcctcg ggcctaccgc tggtttcgta tgcggctgga cctactggtt ctgctggatt 360

gtcacaggca tggctgatgt cgtggctatt accggctaca cccaatactg gtggccagat 420

atcgcgctgt ggatacctgg agcattgacg attttattac tgctcgggtt aaatctcgtg 480

gcagtccgcc tctttggtga gttggaattt tggttcgcaa tcatcaagct ggtggctatt 540

actgcgctca tcctcgtcgg cgcggtgatg gttatttcgc gcttccaatc cccagacggc 600

gacattgcgg ctgtttccaa cctgatcgac catggcggct tcttcccgaa cggaatcacg 660

ggcttcctcg ccggattcca gattgctatc ttcgccttcg tcggcgtgga gcttgccggt 720

accgcggctg cagaaaccaa agacccagaa aagaatcttc ctcgcgcgat taactcgatt 780

cctattcgta tcgtcgtgtt ctacatcctg gctcttgcgg tcatcatgat ggttacccca 840

tgggacaagg tccacagtga ctccagccca tttgtgcaga tgtttgccct ggctggactg 900

ccggcggcag caggcattat taacttcgtg gtgctgacat ctgctgcttc atccgcgaac 960

agtggtattt tctccacatc acgcatgctg ttcggtctgg ctcgcgaagg ccaagcgccc 1020

aagcgttggg gcatcttgtc ccgtaaccaa gtcccagccc gcggcctgct gttctcagta 1080

gcgtgcctgg tcccgagcct ggcaatcttg tacgccggcg ccagcgtcat tgacgccttt 1140

acgttgatta ccaccgtgtc ttctgtgctg ttcatggtgg tatggagcct gatcctcgcg 1200

gcgtaccttg tcttccgccg caagttccca gaacgccatg cagcatccaa gttcaaggtc 1260

ccaggcgggg cgtttatgtg ctgggttgtt ctcgctttct tcgcgtttat ggtcgttgta 1320

ctgacctttg aaaatgacac tcggtccgct ttgatggtca ccccaatctg gttcgtgatc 1380

ctcttggccg gctggttcat ctccggcgga gtcaagcgcg ctaggaagtg a 1431

<210> 3
<211> 40
<212> DNA
<213> Artificial Sequence

<220>
<223> primer

<400> 3
agctcggtac ccggggatcc ccaatgccac ctgctgttcc 40

<210> 4
<211> 32
<212> DNA
<213> Artificial Sequence

<220>
<223> primer

<400> 4

ctcgagacta gttcaggcgc agcgctcgga cg                                    32

<210>    5
<211>    32
<212>    DNA
<213>    Artificial Sequence

<220>
<223>    primer

<400>    5
actagtctcg agtggaggac cgggtgctgg tt                                    32

<210>    6
<211>    40
<212>    DNA
<213>    Artificial Sequence

<220>
<223>    primer

<400>    6
ttgcatgcct gcaggtcgac tgatggcctc ccttgaccag                            40

<210>    7
<211>    1606
<212>    DNA
<213>    Artificial Sequence

<220>
<223>    del-purU

<400>    7
agctcggtac ccggggatcc ccaatgccac ctgctgttcc agaaccatcg cgggaaactc      60

atccaacagc gcctgggcaa cactcacgca gcgcctcccc ttcaccacat gcacctctgg     120

cattttgtgg cgcacgactt ttcccaaaag gacagtcaca tggctcgctc ccctcgggac     180

gctaatttcc gccgttcgag gttcatccaa cagggagtat cctctcacaa tcagtgcagt     240

gaatcccctg attacaccgt tgggaaatct ccgcaatagc gcatctgcca ccacaaccgg     300

attttccgca tattccttct taatccaaaa cgttcccact actcttgtgt actttgcaga     360

gaactgtctt ctattcattt catgctggac atcttcaaat ctaattggct tcatgcttaa     420

gttagactgt gattccgccg attcggttcc ctcccgttta aggcgcccaa ttctccaaat     480

gagctctagt gtcagttcct cgccttcaag ctcttaaaac ggcacacaaa gcatcaaata     540

ttttgcatgc aaggctattt ttcagcccct ttcaccccac cccaggagtc tgtgagttcc     600

ctgatcaact actcaccaca acgagcagcc tcgcagcaga atcggtctga tttttgcatc     660

ccaaggcctc taaagcggga aaatccacca cagatctcaa cattcctgca ggttgtggtg     720

gatttttgca ttgccccgaa gcccgacccc cgaatcgaac ttcgaaccct cttaaccgcc 780

ctcaattacc catcagtccc gattagaacg ccaaactact ccggcgcacc ctccaaaatg 840

gctcccgcat tagaagttcc aatgcgagta gcccctgctt caacaaacgc caccgcatct 900

tcccaagttt tcaccccacc ggcagcctta attccaaccc ttccccgaga agccgaagcc 960

atcacccgaa cagcctcaac agttgcccct cccgctgggt ggaatcccgt ggaagtttta 1020

gcgaagtcag caccagcagc aatcaacgca ttcactgcag tcacaattgc ttcatcactc 1080

acaacagctg tttcgaggat gaatttcagc accacaggag atggaacagc ctccctgatt 1140

gccacaattt cctgcagcaa cctattggca tctccctctt tcactaccgc aatatccaaa 1200

acaacatcta cttcggaagc tccggactgt acggcaaggc gcgcttcggc ggctttcacc 1260

aacgcggggg ttttgccgtg cgggaatcct gcgacggtgg ccactcgaat tccagcttct 1320

tgggcttttg cagttaggtt gaccatgctg ttggggacac agatcgtgcc gactcccagc 1380

tcaattgcgg aatctataaa tgcggccagt tcggagttgg ttacttctgg tccgaggagg 1440

gtgtagtcaa ggatttgggc catggtggag cgggaaatcg tcatatccat accctactta 1500

gacctgactt agtgtgggaa aatttccagg gtagaatgca acgaatgacc ccgagttctc 1560

ctgaagttcg taatcgtccg agcgctgcgc ctgaactagt ctcgag 1606

<210> 8
<211> 1625
<212> DNA
<213> Artificial Sequence

<220>
<223> del-purU

<400> 8
actagtctcg agtggaggac cgggtgctgg tttacggtaa ccgcacggtt gtctttgatt 60

aaggcttttt gcttttcgac gcgaccctaa acaaatgaga cctacccgag ggtaggtctc 120

atttgttttg tgtttagtct gtggtggttt cgcgtagttt ttcttgctta ataaacagca 180

agagaagcag cgcgatgccg atcagtggca tcatcacgta gaacactggg atgagtgcgt 240

cgttgtagga accggcgaac gcatcgtgga gcgcggttgg caattgattg acgattgccg 300

gcgtcaattc gttggagtcc agtccgcctt gggctgccat ggcggcttgt tcttctggtg 360

aaagttgtgc catggctgct ggcattcttt cttccatgag ggttcccaag ttgccaacga 420

acatgccacc gaccagcgcg gatccgagtg aggaaccgat ttgacggaag aagttgttca 480

cagcggttgc ggatccgacc accgcggttg gcagggtgtt ctgaacgatc aggaccagaa 540

cctgcatggc tagacccagg ccgacgccga ggacgaagag gtagattccg atctgccaca 600

gggtggtgga cacttccatt cgggcaaaga agatcagtgc cacgaaggta accaccatgc 660

cgattggtgg gaagagtttg tactttcctg tgttggagat gcggatacca gtccagatgg 720

aggtacccat catgccgacc atcattggga tcagcatgta gccggcttcg gtggcgttga 780

ttccgtggac catctggagg taggtcggaa ggtagccgat gatgccgaac attgcgatac 840

ccaggatcag gcctgcaatg gtggtgagcg tgaagttgcg gttttggaag aaggacattg 900

gaaccaatgg atcttttgcg cgcagttcca caacaaccag cagtgcagcg gcaacgatgg 960

tggtgatgat cagtccaatg atgattggat cagaccactc gtactgggat ccaccccagg 1020

tggtgaacag gatcaggctg gttgcggcaa cgatcatgaa gaaagtgccc aggtaatccc 1080

agcggaactt gacgctcttc tttggaatgt ccaggaagta aatggcgaca ccgatggcga 1140

tgattcccag tgggatgttc atccagaatg cccagcgcca gcctggtcct tcggtgaacc 1200

agccaccgag tagtgggcca agaactgcag agagtccgaa gactccaccc atgacaccca 1260

tgtagcggcc acgttcacgt gctggaacaa cgtccgcgat gattgcctgc gagaggatca 1320

tcagtccacc gccaccgatg ccctgtacgg cacggccgac gatcaaccag gtcatatttc 1380

ctgcaagccc gccgatgacg gagccgatca ggaagatgcc gagggctccg atgaagagac 1440

ctttacgtcc aaccaggtcg ccgagctttc cgtagatcgg cagcatgatg gtttcggcaa 1500

gtaggtatgc ggtgatgacc cacatcatgt ggtcaacgcc gccgagttca ccgacgattg 1560

ttggcagggc tgtgccgaaa atcatctggt caagggaggc catcagtcga cctgcaggca 1620

tgcaa 1625

<210> 9
<211> 40
<212> DNA
<213> Artificial Sequence

<220>
<223> primer

<400> 9
agcgctgcgc ctgaactagt tgccacggtg tttacttaca 40

<210> 10
<211> 40
<212> DNA
<213> Artificial Sequence

<220>
<223> primer

<400> 10
gtcctccact cgagactagt actatctcga agttgtcagc 40

<210> 11
<211> 1970

<212>    DNA
<213>    Artificial Sequence

<220>
<223>    Pn-cycA(cam)

<400>    11
agcgctgcgc ctgaactagt tgccacggtg tttacttaca acggcgatac aattgaaccg      60

caaatggctg ctgagattgc ggaactatcc tgggtatcgc cggatcagcc tgatgttgtg     120

cttgcgcctt tgctcgctga ttttgtcttt cccgccatta attccaaggt gtcttgacgg     180

cactcttatc tccgaacacg cgaagcctag tgtctccaca aaggggttgg agagctagtg     240

gtagtttttc tatcaaatag taggtacgtg cgagagcgcg cagtgacgct ctaccattta     300

agctcctact ttcccattag caagacaggt agattatggc atcttatgat cccgggcatt     360

cacaggttaa aaatacagga gtagaactcg actctcacgt ggagtcagac catctccaac     420

gcggtcttag caaccgacat atccagttga ttgccattgg cggtgctatc ggcaccggac     480

tatttatggg atcgggcaaa accatctccc ttgctggtcc ttccgtaatc ttggtgtatg     540

gcatcatcgg ttttatgctc ttcttcgtca tgcgggcgat gggtgagctt ttgctctcga     600

acctcaacta caagtcattg cgtgatgccg tctcggacat cctcgggcct accgctggtt     660

tcgtatgcgg ctggacctac tggttctgct ggattgtcac aggcatggct gatgtcgtgg     720

ctattaccgg ctacacccaa tactggtggc cagatatcgc gctgtggata cctggagcat     780

tgacgatttt attactgctc gggttaaatc tcgtggcagt ccgcctcttt ggtgagttgg     840

aattttggtt cgcaatcatc aagctggtgg ctattactgc gctcatcctc gtcggcgcgg     900

tgatggttat ttcgcgcttc caatccccag acggcgacat tgcggctgtt tccaacctga     960

tcgaccatgg cggcttcttc ccgaacggaa tcacgggctt cctcgccgga ttccagattg    1020

ctatcttcgc cttcgtcggc gtggagcttg ccggtaccgc ggctgcagaa accaaagacc    1080

cagaaaagaa tcttcctcgc gcgattaact cgattcctat tcgtatcgtc gtgttctaca    1140

tcctggctct tgcggtcatc atgatggtta ccccatggga caaggtccac agtgactcca    1200

gcccatttgt gcagatgttt gccctggctg gactgccggc ggcagcaggc attattaact    1260

tcgtggtgct gacatctgct gcttcatccg cgaacagtgg tattttctcc acatcacgca    1320

tgctgttcgg tctggctcgc gaaggccaag cgcccaagcg ttggggcatc ttgtcccgta    1380

accaagtccc agcccgcggc ctgctgttct cagtagcgtg cctggtcccg agcctggcaa    1440

tcttgtacgc cggcgccagc gtcattgacg cctttacgtt gattaccacc gtgtcttctg    1500

tgctgttcat ggtggtatgg agcctgatcc tcgcggcgta ccttgtcttc cgccgcaagt    1560

tcccagaacg ccatgcagca tccaagttca aggtcccagg cggggcgttt atgtgctggg    1620

ttgttctcgc tttcttcgcg tttatggtcg ttgtactgac ctttgaaaat gacactcggt    1680

ccgctttgat ggtcacccca atctggttcg tgatcctctt ggccggctgg ttcatctccg 1740

gcggagtcaa gcgcgctagg aagtgagcgc aacaaagccc cgccacagaa aaacgcgagg 1800

acttcgtgca ttaagacgaa atatattaga tgtccatgtt gtctatcaga cgggtgtcac 1860

cgacggagat agcgcccagt gctagagcag gagcggttgg aggccacgca atcggtttga 1920

gcgccttagg gctgacaact tcgagatagt actagtctcg agtggaggac 1970

<210> 12
<211> 40
<212> DNA
<213> Artificial Sequence

<220>
<223> primer

<400> 12
agcgctgcgc ctgaactagt agaaacatcc cagcgctact 40

<210> 13
<211> 32
<212> DNA
<213> Artificial Sequence

<220>
<223> primer

<400> 13
ataagatgcc atgagtgttt cctttcgttg gg 32

<210> 14
<211> 32
<212> DNA
<213> Artificial Sequence

<220>
<223> primer

<400> 14
aaggaaacac tcatggcatc ttatgatccc gg 32

<210> 15
<211> 1964
<212> DNA
<213> Artificial Sequence

<220>
<223> pcj7-cycA(Cam)

<400> 15
agcgctgcgc ctgaactagt ccagcgctac taatagggag cgttgacctt ccttccacgg 60

accggtaatc ggagtgccta aaaccgcatg cggcttaggc tccaagatag gttctgcgcg 120

gccgggtaat gcatcttctt tagcaacaag ttgaggggta ggtgcaaata agaacgacat 180

agaaatcgtc tcctttctgt ttttaatcaa catacaccac cacctaaaaa ttccccgacc 240

agcaagttca cagtattcgg gcacaatatc gttgccaaaa tattgtttcg gaatatcatg 300

ggatacgtac ccaacgaaag gaaacactca tggcatctta tgatcccggg cattcacagg 360

ttaaaaatac aggagtagaa ctcgactctc acgtggagtc agaccatctc caacgcggtc 420

ttagcaaccg acatatccag ttgattgcca ttggcggtgc tatcggcacc ggactattta 480

tgggatcggg caaaaccatc tcccttgctg gtccttccgt aatcttggtg tatggcatca 540

tcggttttat gctcttcttc gtcatgcggg cgatgggtga gcttttgctc tcgaacctca 600

actacaagtc attgcgtgat gccgtctcgg acatcctcgg gcctaccgct ggtttcgtat 660

gcggctggac ctactggttc tgctggattg tcacaggcat ggctgatgtc gtggctatta 720

ccggctacac ccaatactgg tggccagata tcgcgctgtg gatacctgga gcattgacga 780

ttttattact gctcgggtta aatctcgtgg cagtccgcct ctttggtgag ttggaatttt 840

ggttcgcaat catcaagctg gtggctatta ctgcgctcat cctcgtcggc gcggtgatgg 900

ttatttcgcg cttccaatcc ccagacggcg acattgcggc tgtttccaac ctgatcgacc 960

atggcggctt cttcccgaac ggaatcacgg gcttcctcgc cggattccag attgctatct 1020

tcgccttcgt cggcgtggag cttgccggta ccgcggctgc agaaaccaaa gacccagaaa 1080

agaatcttcc tcgcgcgatt aactcgattc ctattcgtat cgtcgtgttc tacatcctgg 1140

ctcttgcggt catcatgatg gttaccccat gggacaaggt ccacagtgac tccagcccat 1200

ttgtgcagat gtttgccctg gctggactgc cggcggcagc aggcattatt aacttcgtgg 1260

tgctgacatc tgctgcttca tccgcgaaca gtggtatttt ctccacatca cgcatgctgt 1320

tcggtctggc tcgcgaaggc caagcgccca agcgttgggg catcttgtcc cgtaaccaag 1380

tcccagcccg cggcctgctg ttctcagtag cgtgcctggt cccgagcctg gcaatcttgt 1440

acgccggcgc cagcgtcatt gacgccttta cgttgattac caccgtgtct tctgtgctgt 1500

tcatggtggt atggagcctg atcctcgcgg cgtaccttgt cttccgccgc aagttcccag 1560

aacgccatgc agcatccaag ttcaaggtcc caggcggggc gtttatgtgc tgggttgttc 1620

tcgctttctt cgcgtttatg gtcgttgtac tgacctttga aaatgacact cggtccgctt 1680

tgatggtcac cccaatctgg ttcgtgatcc tcttggccgg ctggttcatc tccggcggag 1740

tcaagcgcgc taggaagtga gcgcaacaaa gccccgccac agaaaaacgc gaggacttcg 1800

tgcattaaga cgaaatatat tagatgtcca tgttgtctat cagacgggtg tcaccgacgg 1860

agatagcgcc cagtgctaga gcaggagcgg ttggaggcca cgcaatcggt ttgagcgcct 1920

tagggctgac aacttcgaga tagtactagt ctcgagtgga ggac 1964

<210>    16
<211>    40
<212>    DNA
<213>    Artificial Sequence

<220>
<223>    primer


<400>    16
agcgctgcgc ctgaactagt aaatcaaggt aggggctgaa                              40


<210>    17
<211>    30
<212>    DNA
<213>    Artificial Sequence

<220>
<223>    primer


<400>    17
ataagatgcc atcaggtcag ctaacctttc                                         30


<210>    18
<211>    30
<212>    DNA
<213>    Artificial Sequence

<220>
<223>    primer


<400>    18
ttagctgacc tgatggcatc ttatgatccc                                         30


<210>    19
<211>    1963
<212>    DNA
<213>    Artificial Sequence

<220>
<223>    PglyA-cycA(Cam)


<400>    19
agcgctgcgc ctgaactagt aaatcaaggt aggggctgaa atctggtgtg cgtggggaag        60

gtttcacacc agttgtgctt gcagcgtttt gctctgccat gaatccattg tgcaccttag       120

ctactccact agtgtgatcg gggttatttt ttcacttcaa tgggtggcta aaagacgtgg       180

gcacgtgagt aaactcatgc gcgcgaaacg atgggagtga acccatactt ttatatatgg       240

gtatcggcgg tctatgcttg tgggcgtacc tgtcccgcga gtgaggtctt acgcgcggga       300

ttcgtcttgt gaaaggttag ctgacctgat ggcatcttat gatcccgggc attcacaggt       360

taaaaataca ggagtagaac tcgactctca cgtggagtca gaccatctcc aacgcggtct 420

tagcaaccga catatccagt tgattgccat tggcggtgct atcggcaccg gactatttat 480

gggatcgggc aaaaccatct cccttgctgg tccttccgta atcttggtgt atggcatcat 540

cggttttatg ctcttcttcg tcatgcgggc gatgggtgag cttttgctct cgaacctcaa 600

ctacaagtca ttgcgtgatg ccgtctcgga catcctcggg cctaccgctg gtttcgtatg 660

cggctggacc tactggttct gctggattgt cacaggcatg gctgatgtcg tggctattac 720

cggctacacc caatactggt ggccagatat cgcgctgtgg atacctggag cattgacgat 780

tttattactg ctcgggttaa atctcgtggc agtccgcctc tttggtgagt tggaattttg 840

gttcgcaatc atcaagctgg tggctattac tgcgctcatc ctcgtcggcg cggtgatggt 900

tatttcgcgc ttccaatccc cagacggcga cattgcggct gtttccaacc tgatcgacca 960

tggcggcttc ttcccgaacg gaatcacggg cttcctcgcc ggattccaga ttgctatctt 1020

cgccttcgtc ggcgtggagc ttgccggtac cgcggctgca gaaaccaaag acccagaaaa 1080

gaatcttcct cgcgcgatta actcgattcc tattcgtatc gtcgtgttct acatcctggc 1140

tcttgcggtc atcatgatgg ttaccccatg ggacaaggtc cacagtgact ccagcccatt 1200

tgtgcagatg tttgccctgg ctggactgcc ggcggcagca ggcattatta acttcgtggt 1260

gctgacatct gctgcttcat ccgcgaacag tggtattttc tccacatcac gcatgctgtt 1320

cggtctggct cgcgaaggcc aagcgcccaa gcgttggggc atcttgtccc gtaaccaagt 1380

cccagcccgc ggcctgctgt tctcagtagc gtgcctggtc ccgagcctgg caatcttgta 1440

cgccggcgcc agcgtcattg acgcctttac gttgattacc accgtgtctt ctgtgctgtt 1500

catggtggta tggagcctga tcctcgcggc gtaccttgtc ttccgccgca agttcccaga 1560

acgccatgca gcatccaagt tcaaggtccc aggcggggcg tttatgtgct gggttgttct 1620

cgctttcttc gcgtttatgg tcgttgtact gacctttgaa aatgacactc ggtccgcttt 1680

gatggtcacc ccaatctggt tcgtgatcct cttggccggc tggttcatct ccggcggagt 1740

caagcgcgct aggaagtgag cgcaacaaag ccccgccaca gaaaaacgcg aggacttcgt 1800

gcattaagac gaaatatatt agatgtccat gttgtctatc agacgggtgt caccgacgga 1860

gatagcgccc agtgctagag caggagcggt tggaggccac gcaatcggtt tgagcgcctt 1920

agggctgaca acttcgagat agtactagtc tcgagtggag gac 1963

<210> 20
<211> 470
<212> PRT
<213> Escherichia coli

<400> 20
Met Val Asp Gln Val Lys Val Val Ala Asp Asp Gln Ala Pro Ala Glu
1               5                   10                  15

Gln Ser Leu Arg Arg Asn Leu Thr Asn Arg His Ile Gln Leu Ile Ala
            20                  25                  30

Ile Gly Gly Ala Ile Gly Thr Gly Leu Phe Met Gly Ser Gly Lys Thr
        35                  40                  45

Ile Ser Leu Ala Gly Pro Ser Ile Ile Phe Val Tyr Met Ile Ile Gly
    50                  55                  60

Phe Met Leu Phe Phe Val Met Arg Ala Met Gly Glu Leu Leu Leu Ser
65                  70                  75                  80

Asn Leu Glu Tyr Lys Ser Phe Ser Asp Phe Ala Ser Asp Leu Leu Gly
                85                  90                  95

Pro Trp Ala Gly Tyr Phe Thr Gly Trp Thr Tyr Trp Phe Cys Trp Val
            100                 105                 110

Val Thr Gly Met Ala Asp Val Val Ala Ile Thr Ala Tyr Ala Gln Phe
        115                 120                 125

Trp Phe Pro Asp Leu Ser Asp Trp Val Ala Ser Leu Ala Val Ile Val
    130                 135                 140

Leu Leu Leu Thr Leu Asn Leu Ala Thr Val Lys Met Phe Gly Glu Met
145                 150                 155                 160

Glu Phe Trp Phe Ala Met Ile Lys Ile Val Ala Ile Val Ser Leu Ile
                165                 170                 175

Val Val Gly Leu Val Met Val Ala Met His Phe Gln Ser Pro Thr Gly
            180                 185                 190

Val Glu Ala Ser Phe Ala His Leu Trp Asn Asp Gly Gly Trp Phe Pro
        195                 200                 205

Lys Gly Leu Ser Gly Phe Phe Ala Gly Phe Gln Ile Ala Val Phe Ala
    210                 215                 220

Phe Val Gly Ile Glu Leu Val Gly Thr Thr Ala Ala Glu Thr Lys Asp
225                 230                 235                 240

Pro Glu Lys Ser Leu Pro Arg Ala Ile Asn Ser Ile Pro Ile Arg Ile
                245                 250                 255

Ile Met Phe Tyr Val Phe Ala Leu Ile Val Ile Met Ser Val Thr Pro
            260                 265                 270

Trp Ser Ser Val Val Pro Glu Lys Ser Pro Phe Val Glu Leu Phe Val
        275                 280                 285

Leu Val Gly Leu Pro Ala Ala Ala Ser Val Ile Asn Phe Val Val Leu
    290                 295                 300

Thr Ser Ala Ala Ser Ser Ala Asn Ser Gly Val Phe Ser Thr Ser Arg
305                 310                 315                 320

Met Leu Phe Gly Leu Ala Gln Glu Gly Val Ala Pro Lys Ala Phe Ala
                325                 330                 335

Lys Leu Ser Lys Arg Ala Val Pro Ala Lys Gly Leu Thr Phe Ser Cys
            340                 345                 350

Ile Cys Leu Leu Gly Gly Val Val Met Leu Tyr Val Asn Pro Ser Val
        355                 360                 365

Ile Gly Ala Phe Thr Met Ile Thr Thr Val Ser Ala Ile Leu Phe Met
    370                 375                 380

Phe Val Trp Thr Ile Ile Leu Cys Ser Tyr Leu Val Tyr Arg Lys Gln
385                 390                 395                 400

Arg Pro His Leu His Glu Lys Ser Ile Tyr Lys Met Pro Leu Gly Lys
                405                 410                 415

Leu Met Cys Trp Val Cys Met Ala Phe Phe Val Phe Val Val Val Leu
            420                 425                 430

Leu Thr Leu Glu Asp Asp Thr Arg Gln Ala Leu Leu Val Thr Pro Leu
        435                 440                 445

Trp Phe Ile Ala Leu Gly Leu Gly Trp Leu Phe Ile Gly Lys Lys Arg
    450                 455                 460

Ala Ala Glu Leu Arg Lys
465                 470

<210>    21
<211>    1413
<212>    DNA
<213>    Escherichia coli

<400>    21
atggtagatc aggtaaaagt cgttgccgat gatcaggctc cggctgaaca gtcgctacgg      60

cgcaatctca caaaccgaca tattcagctt attgccattg gcggtgccat tggtacgggg     120

ttgtttatgg ggtctggcaa aacgattagc cttgccgggc cgtcgatcat tttcgtttat     180

atgatcattg gttttatgct ctttttcgtg atgcgggcaa tgggggaatt gctgctttcg     240

aatctggaat acaaatcttt tagtgacttc gcttccgatt tactcgggcc gtgggcagga     300

tatttcaccg gctggactta ctggttctgc tgggttgtaa ccggtatggc agacgtggtg     360

gcgatcacgg cttatgctca gttctggttc cccgatctct ccgactgggt cgcctcgctg     420

gcggtgatag tgctgctgct gacgctcaat ctcgccaccg tgaaaatgtt cggtgagatg     480

gagttctggt ttgcgatgat caaaatcgtc gccattgtgt cgctgattgt cgtcggcctg     540

gtcatggtgg cgatgcactt tcagtcaccg actggtgtgg aagcgtcatt cgcgcatttg     600

tggaatgacg gcggctggtt cccgaaaggt ttaagtggct tctttgccgg attccagata     660

gcggttttcg ctttcgtggg gattgagctg gtaggtacaa cagctgcgga aaccaaagat     720

ccagagaaat cactgccacg cgcgattaac tccattccga tccgtatcat tatgttctac     780

gtcttcgcgc tgattgtgat tatgtccgtg acgccgtgga gttcggtagt cccggagaaa     840

agcccgtttg ttgaactgtt cgtgttggta gggctgcctg ctgccgcaag cgtgatcaac     900

tttgtggtgc tgacctctgc ggcgtcttcc gctaacagcg gcgtcttctc taccagccgt     960

atgctgtttg gtctggcgca ggaaggtgtg gcaccgaaag cgttcgctaa actttctaag 1020

cgcgcagtac ccgcgaaagg gctgacgttc tcgtgtatct gtctgctcgg cggcgtggtg 1080

atgttgtatg tgaatcctag tgtgattggc gcgttcacga tgattacaac cgtttccgcg 1140

attctgttta tgttcgtctg gacgattatc ctttgctcgt accttgtgta tcgcaaacag 1200

cgtcctcatc tacatgagaa gtcgatctac aagatgccgc tcggcaagct gatgtgctgg 1260

gtatgtatgg cgttctttgt gttcgtggtc gtgttgctga cactggaaga tgacactcgc 1320

caggcgctgc tggttacccc gctgtggttt atcgcgctgg ggttgggctg gctgtttatt 1380

ggtaagaagc gggctgctga actgcggaaa taa 1413

<210> 22
<211> 32
<212> DNA
<213> Artificial Sequence

<220>
<223> primer

<400> 22
ctgatctacc atgagtgttt cctttcgttg gg 32

<210> 23
<211> 32
<212> DNA
<213> Artificial Sequence

<220>
<223> primer

<400> 23
aaggaaacac tcatggtaga tcaggtaaaa gt 32

<210> 24
<211> 40
<212> DNA
<213> Artificial Sequence

<220>
<223> primer

<400> 24
gtcctccact cgagactagt aactgctgga agtgattaaa 40

<210> 25
<211> 1985
<212> DNA
<213> Artificial Sequence

<220>

<223>    pcj7-cycA(Eco)

<400>    25
agcgctgcgc ctgaactagt agaaacatcc cagcgctact aatagggagc gttgaccttc    60

cttccacgga ccggtaatcg gagtgcctaa aaccgcatgc ggcttaggct ccaagatagg    120

ttctgcgcgg ccgggtaatg catcttcttt agcaacaagt tgaggggtag gtgcaaataa    180

gaacgacata gaaatcgtct cctttctgtt tttaatcaac atacaccacc acctaaaaat    240

tccccgacca gcaagttcac agtattcggg cacaatatcg ttgccaaaat attgtttcgg    300

aatatcatgg gatacgtacc caacgaaagg aaacactcat ggtagatcag gtaaaagtcg    360

ttgccgatga tcaggctccg gctgaacagt cgctacggcg caatctcaca aaccgacata    420

ttcagcttat tgccattggc ggtgccattg gtacggggtt gtttatgggg tctggcaaaa    480

cgattagcct tgccgggccg tcgatcattt tcgtttatat gatcattggt tttatgctct    540

ttttcgtgat gcgggcaatg ggggaattgc tgctttcgaa tctggaatac aaatctttta    600

gtgacttcgc ttccgattta ctcgggccgt gggcaggata tttcaccggc tggacttact    660

ggttctgctg ggttgtaacc ggtatggcag acgtggtggc gatcacggct tatgctcagt    720

tctggttccc cgatctctcc gactgggtcg cctcgctggc ggtgatagtg ctgctgctga    780

cgctcaatct cgccaccgtg aaaatgttcg gtgagatgga gttctggttt gcgatgatca    840

aaatcgtcgc cattgtgtcg ctgattgtcg tcggcctggt catggtggcg atgcactttc    900

agtcaccgac tggtgtggaa gcgtcattcg cgcatttgtg gaatgacggc ggctggttcc    960

cgaaaggttt aagtggcttc tttgccggat tccagatagc ggttttcgct ttcgtgggga    1020

ttgagctggt aggtacaaca gctgcggaaa ccaaagatcc agagaaatca ctgccacgcg    1080

cgattaactc cattccgatc cgtatcatta tgttctacgt cttcgcgctg attgtgatta    1140

tgtccgtgac gccgtggagt tcggtagtcc cggagaaaag cccgtttgtt gaactgttcg    1200

tgttggtagg gctgcctgct gccgcaagcg tgatcaactt tgtggtgctg acctctgcgg    1260

cgtcttccgc taacagcggc gtcttctcta ccagccgtat gctgtttggt ctggcgcagg    1320

aaggtgtggc accgaaagcg ttcgctaaac tttctaagcg cgcagtaccc gcgaaagggc    1380

tgacgttctc gtgtatctgt ctgctcggcg gcgtggtgat gttgtatgtg aatcctagtg    1440

tgattggcgc gttcacgatg attacaaccg tttccgcgat tctgtttatg ttcgtctgga    1500

cgattatcct ttgctcgtac cttgtgtatc gcaaacagcg tcctcatcta catgagaagt    1560

cgatctacaa gatgccgctc ggcaagctga tgtgctgggt atgtatggcg ttctttgtgt    1620

tcgtggtcgt gttgctgaca ctggaagatg acactcgcca ggcgctgctg gttaccccgc    1680

tgtggtttat cgcgctgggg ttgggctggc tgtttattgg taagaagcgg gctgctgaac    1740

tgcggaaata accgcattat catgctggat ggcgcaatgc catccagctt ttagatcact    1800

cacccgccag cgcgcgtggg aacagtacat tgttttccag actgatgtga tccatcaggt 1860

catcaatcag ttcattaatg ccgttataca tcgctttcca ggtggtgcag gcttctggcg 1920

gcggtgtgac gttattggtg gtgtgtttaa tcacttccag cagttactag tctcgagtgg 1980

aggac 1985

<210> 26
<211> 952
<212> PRT
<213> Corynebacterium ammoniagenes

<400> 26
   Met Asp Phe Ile Ala Arg His Leu Gly Pro Asp Ala Thr Glu Ser Lys
   1 5 10 15
   Asp Met Leu Ala Arg Val Gly Tyr Asp Ser Val Glu Ala Leu Val Thr
   20 25 30
   Ser Ala Ile Pro Gln Ser Ile Ser Ile Thr Asp Ala Leu Asn Met Pro
   35 40 45
   Gln Ala Leu Ser Glu Thr Asp Ala Gln Ala Lys Leu Arg Ala Tyr Ala
   50 55 60
   Asp Lys Asn Val Val Leu Lys Ser Phe Tyr Gly Gln Gly Tyr Ser Asp
   65 70 75 80
   Thr Ile Thr Pro Ala Val Ile Arg Arg Gly Leu Val Glu Asp Ala Gly
   85 90 95
   Trp Tyr Thr Ala Tyr Thr Pro Tyr Gln Pro Glu Ile Ser Gln Gly Arg
   100 105 110
   Leu Glu Ser Leu Leu Asn Phe Gln Thr Met Val Gln Asp Leu Thr Gly
   115 120 125
   Leu Pro Ile Ala Asn Ala Ser Leu Leu Asp Glu Ala Ser Ala Val Ala
   130 135 140
   Glu Ala Val Gly Leu Met Ser Arg Ala Val Lys Lys Gly Arg Arg Val
   145 150 155 160
   Leu Leu Asp Ala Arg Leu His Pro Gln Val Leu Thr Val Ala Ala Glu
   165 170 175
   Arg Ala Arg Ala Ile Asp Leu Glu Val Glu Ile Ala Asp Leu Ser Asn
   180 185 190
   Gly Val Val Gly Glu Asp Leu Val Gly Ala Val Val Ala Tyr Thr Gly
   195 200 205
   Thr Glu Gly Asp Ile Phe Asp Pro Arg Ala Val Ile Glu Glu Ile His
   210 215 220
   Gly Arg Gly Gly Leu Val Ser Val Ala Ala Asp Leu Leu Ser Leu Leu
   225 230 235 240
   Leu Leu Glu Gly Pro Gly Ser Phe Gly Ala Asp Ile Val Ile Gly Ser
   245 250 255
   Ser Gln Arg Phe Gly Val Pro Leu Phe Phe Gly Gly Pro His Ala Ala
               260                 265                 270
   Phe Met Ala Val Thr Asp Lys Leu Lys Arg Gln Met Pro Gly Arg Leu
           275                 280                 285
   Val Gly Val Ser Val Asp Ser Glu Gly Arg Pro Ala Tyr Arg Leu Ala
       290                 295                 300
   Leu Gln Thr Arg Glu Gln His Ile Arg Arg Glu Arg Ala Thr Ser Asn
   305                 310                 315                 320
   Ile Cys Thr Ala Gln Ala Leu Leu Ala Asn Val Ala Ala Met Tyr Ala
                   325                 330                 335
   Val Tyr His Gly Pro Glu Gly Leu Lys Glu Ile Ala Asn His Val His
               340                 345                 350
   Ser Leu Ala Ala Ser Phe Ala Gly Ala Val Thr Thr Gln Gly Leu Lys
           355                 360                 365
   Ile Thr Ser Ser Glu Phe Phe Asp Thr Val Thr Val Ala Gly Val Asp
       370                 375                 380
   Ala Ala Ser Ile Lys Phe Ser Leu Glu Lys Ala Gly Tyr Leu Val Arg
   385                 390                 395                 400
   Thr Ile Gly Glu Asp Lys Val Ser Val Ser Phe Gly Glu Ser Ala Thr
                   405                 410                 415
   Gln Gly Asp Val Thr Val Leu Ala Asp Ala Phe Gly Ala Ala Ala Val
               420                 425                 430
   Asp Asn Ala Asp Phe Pro Leu Pro Glu Ala Leu Thr Arg Thr Thr Glu
           435                 440                 445
   Val Leu Thr His Glu Ile Phe Asn Ser Ile His Ser Glu Thr Gln Met
       450                 455                 460
   Met Arg Tyr Leu Arg Lys Leu Gly Asp Lys Asp Leu Ala Leu Asp Arg
   465                 470                 475                 480
   Thr Met Ile Pro Leu Gly Ser Cys Thr Met Lys Leu Asn Pro Thr Ala
                   485                 490                 495
   Ala Met Glu Pro Ile Thr Trp Pro Glu Phe Ala Asn Val His Pro Tyr
               500                 505                 510
   Ser Pro Glu Tyr Ala Thr Gln Gly Trp Arg Glu Leu Ile Glu Glu Leu
           515                 520                 525
   Glu Gly Trp Leu Ala Glu Leu Thr Gly Tyr Ala Lys Val Ser Ile Gln
       530                 535                 540
   Pro Asn Ala Gly Ser Gln Gly Glu Leu Ala Gly Leu Leu Ala Ile Arg
   545                 550                 555                 560
   Arg Tyr His Val Ala Asn Gly Asp Thr Asn Arg Asp Ile Val Leu Ile
                   565                 570                 575
   Pro Ala Ser Ala His Gly Thr Asn Ala Ala Ser Ala Thr Leu Ala Asn
               580                 585                 590
   Leu Arg Val Val Val Val Lys Thr Ala Glu Asp Gly Ser Ile Asp Leu
           595                 600                 605
   Glu Asp Leu Asp Ala Lys Ile Ala Lys His Gly Gln Asn Met Ala Gly
       610                 615                 620
   Ile Met Ile Thr Tyr Pro Ser Thr His Gly Val Phe Asp Pro Glu Val
   625                 630                 635                 640
   Arg Glu Val Cys Asp Lys Ile His Ala Ala Gly Gly Gln Val Tyr Ile
                   645                 650                 655
   Asp Gly Ala Asn Met Asn Ala Leu Thr Gly Trp Ala Gln Pro Gly Lys
               660                 665                 670
   Phe Gly Gly Asp Val Ser His Leu Asn Leu His Lys Thr Phe Thr Ile
           675                 680                 685
   Pro His Gly Gly Gly Gly Pro Gly Val Gly Pro Ile Gly Val Ala Glu
       690                 695                 700
   His Leu Ile Pro Phe Leu Pro Thr Asp Ala Ala Ala Asp Glu Leu Asp
   705                 710                 715                 720
   Pro Ala Asn Pro Thr Pro Val Glu Gln Gly Val Pro Ile Thr Ala Ser
                   725                 730                 735
   Gln Phe Gly Ser Ala Gly Val Leu Pro Ile Thr Trp Ala Tyr Ile Ala
               740                 745                 750
   Met Thr Gly Gly Glu Gly Leu Thr Ser Ala Thr Ala His Ala Ile Leu
           755                 760                 765
   Gly Ala Asn Tyr Leu Ala Arg Glu Leu Ser Asp Ser Phe Pro Ile Leu
       770                 775                 780
   Phe Thr Gly Asn Glu Gly Leu Val Ala His Glu Cys Ile Leu Asp Leu
   785                 790                 795                 800
   Arg Ala Leu Thr Asp Ala Ser Gly Val Thr Ala Ala Asp Val Ala Lys
                   805                 810                 815
   Arg Leu Ile Asp Phe Gly Phe His Ala Pro Thr Leu Ala Phe Pro Val
               820                 825                 830
   Ala Gly Thr Leu Met Val Glu Pro Thr Glu Ser Glu Asp Ile Ala Glu
           835                 840                 845
   Leu Asp Arg Phe Ile Glu Ala Met Arg Thr Ile Arg Ala Glu Ile Gln
       850                 855                 860
   Glu Ile Ile Asp Gly Lys Ile Ala Tyr Glu Asp Ser Val Ile Arg His
   865                 870                 875                 880
   Ala Pro Tyr Thr Ala Pro Ser Val Ser Ser Asp Asp Trp Glu Tyr Ser
                   885                 890                 895
   Phe Ser Arg Glu Lys Ala Ala Trp Pro Val Pro Ser Leu Arg Leu Asn
               900                 905                 910
   Lys Tyr Phe Pro Pro Val Arg Arg Leu Asp Glu Ala Tyr Gly Asp Arg
           915                 920                 925
   Asn Leu Val Cys Ser Cys Pro Pro Pro Glu Ala Phe Asp Phe Asp Ala
       930                 935                 940
   Asp Thr Asp Ser Thr Glu Glu Ala
   945                 950


<210>    27
<211>    367
<212>    PRT
<213>    Corynebacterium ammoniagenes

<400>    27
Met Ser Glu Leu Arg Gln Ser Pro Leu His Ala Glu His Glu Lys Leu
  1               5                  10                  15

Gly Ala Ser Phe Thr Ala Phe Gly Pro Trp Asn Met Pro Leu Lys Tyr
             20                  25                  30

Gly Lys Glu Leu Asp Glu His His Ala Val Arg Asn Ala Val Gly Met
         35                  40                  45

Phe Asp Leu Ser His Met Gly Glu Ile Trp Val Asn Gly Pro Asp Ala
     50                  55                  60

Ala Ala Phe Leu Ser Tyr Ala Leu Ile Ser Asn Met Glu Thr Val Lys
 65                  70                  75                  80

Asn Gly Lys Ala Lys Tyr Ser Met Ile Val Ala Glu Asp Gly Gly Ile
                 85                  90                  95

Ile Asp Asp Leu Ile Ser Tyr Arg Phe Ser Asp Thr Lys Phe Leu Val
            100                 105                 110

Val Pro Asn Ala Gly Asn Thr Asp Val Val Trp Glu Ala Phe Asn Gln
        115                 120                 125

Arg Ile Glu Gly Phe Asp Val Glu Leu Asn Asn Glu Ser Leu Asp Val
    130                 135                 140

Ala Met Ile Ala Leu Gln Gly Pro Asn Ala Ala Lys Val Leu Val Glu
145                 150                 155                 160

Gln Val Ala Glu Glu Ser Lys Glu Glu Val Glu Asn Leu Pro Tyr Tyr
                165                 170                 175

Ala Ala Thr Met Ala Lys Val Ala Asp Val Asp Thr Ile Val Ala Arg
            180                 185                 190

Thr Gly Tyr Thr Gly Glu Asp Gly Phe Glu Leu Met Ile Tyr Asn Ala
        195                 200                 205

Asp Ala Thr Lys Leu Trp Gln Leu Phe Ile Asp Gln Asp Gly Val Thr
    210                 215                 220

Pro Cys Gly Leu Ala Ser Arg Asp Ser Leu Arg Leu Glu Ala Gly Met
225                 230                 235                 240

Pro Leu Tyr Gly Asn Glu Leu Ser Arg Asp Ile Thr Pro Val Glu Ala
                245                 250                 255

Gly Met Gly Val Ala Phe Lys Lys Lys Thr Ala Asp Phe Val Gly Ala

260                 265                 270

Glu Val Leu Arg Gln Arg Leu Glu Glu Gly Pro Lys Gln Val Ile Lys
        275                 280                 285

Ala Leu Thr Ser Ser Glu Arg Arg Ala Ala Arg Thr Gly Ala Glu Ile
    290                 295                 300

Tyr Ala Gly Glu Gln Leu Val Gly Thr Val Thr Ser Gly Gln Pro Ser
305                 310                 315                 320

Pro Thr Leu Gly His Pro Ile Ala Leu Ala Leu Val Asp Thr Ala Ala
                325                 330                 335

Asn Leu Glu Glu Gly Ala Glu Val Glu Val Asp Ile Arg Gly Lys Arg
            340                 345                 350

Tyr Pro Phe Thr Val Thr Lys Thr Pro Phe Tyr Ser Arg Glu Lys
        355                 360                 365

<210>    28
<211>    129
<212>    PRT
<213>    Corynebacterium ammoniagenes

<400>    28
Met Ala Asn Leu Pro Ala Glu Phe Thr Tyr Ser Glu Asp His Glu Trp
  1               5                  10                  15

Ile Asn Ala Ala Gln Asp Ala Ile Val Gly Lys Thr Val Arg Ile Gly
            20                  25                  30

Ile Thr Ser Val Ala Ala Asp Arg Leu Gly Glu Val Val Phe Ala Glu
        35                  40                  45

Leu Pro Ala Val Gly Asp Ser Val Thr Ala Gly Glu Thr Cys Gly Glu
    50                  55                  60

Val Glu Ser Thr Lys Ser Val Ser Asp Leu Tyr Ser Pro Val Thr Gly
 65                  70                  75                  80

Thr Val Thr Ala Val Asn Glu Thr Val His Asp Asp Tyr Glu Ile Ile
                85                  90                  95

Asn Asn Asp Pro Phe Gly Glu Gly Trp Leu Phe Glu Val Glu Val Glu
            100                 105                 110

Glu Leu Gly Glu Val Met Thr Ala Asp Glu Tyr Ala Ala Glu Asn Gly
        115                 120                 125

Ile

<210>    29
<211>    353
<212>    PRT
<213>    Corynebacterium ammoniagenes

<400>    29

Met Leu Arg Ile Glu Lys Lys Asn Ala Glu Ser Pro Ile Glu Gln Lys
1               5                   10                  15

Pro Arg Trp Ile Arg Asn Gln Val Arg Thr Gly Pro Gly Tyr Glu Asp
            20                  25                  30

Met Lys Lys Arg Val Ala Gly Ala Gly Leu His Thr Val Cys Gln Glu
        35                  40                  45

Ala Gly Cys Pro Asn Ile His Glu Cys Trp Glu Ser Arg Glu Ala Thr
    50                  55                  60

Phe Leu Ile Gly Gly Asp Arg Cys Thr Arg Arg Cys Asp Phe Cys Asp
65                  70                  75                  80

Ile Ala Thr Gly Lys Pro Gln Ala Leu Asp Thr Asp Glu Pro Arg Arg
                85                  90                  95

Val Ser Glu Asn Ile Gln Glu Met Asn Leu Asn Tyr Ala Thr Ile Thr
            100                 105                 110

Gly Val Thr Arg Asp Asp Leu Pro Asp Glu Gly Ala Trp Leu Tyr Ala
        115                 120                 125

Glu Val Val Arg Lys Ile His Glu Lys Asn Pro His Thr Gly Val Glu
    130                 135                 140

Asn Leu Thr Pro Asp Phe Ser Gly Lys Pro Asp Leu Leu Gln Glu Val
145                 150                 155                 160

Phe Glu Ala Arg Pro Glu Val Phe Ala His Asn Leu Glu Thr Val Pro
                165                 170                 175

Arg Ile Phe Lys Arg Ile Arg Pro Ala Phe Arg Tyr Glu Arg Ser Leu
            180                 185                 190

Asp Val Leu Gln Gln Ala His Asp Phe Gly Leu Ile Thr Lys Ser Asn
        195                 200                 205

Leu Ile Leu Gly Met Gly Glu Thr Glu Glu Glu Ile Gln Glu Ala Leu
    210                 215                 220

Arg Asp Met Arg Ser Val Gly Thr Asp Ile Ile Thr Ile Thr Gln Tyr
225                 230                 235                 240

Leu Arg Pro Gly Pro Arg Phe His Pro Ile Glu Arg Trp Val Arg Pro
                245                 250                 255

Glu Glu Phe Ile Ala His Ser Glu Tyr Ala Lys Glu Leu Gly Phe Thr
            260                 265                 270

Val Met Ser Gly Pro Leu Val Arg Ser Ser Tyr Arg Ala Gly Lys Leu
        275                 280                 285

Tyr Thr Gln Ala Met Lys Ala Arg Gly Trp Glu Leu Pro Glu Asn Leu
    290                 295                 300

Lys His Leu Glu Glu Thr Ser Asp Gly Ala Thr Ala Gln Glu Ala Ser
305                 310                 315                 320

Ser Leu Leu Lys Lys Tyr Gly Pro Ser Glu Glu Thr Pro Val Thr Ser
                325                 330                 335

Arg Met Ala Lys Thr Pro Val Gly Ala Asp Lys Phe Thr Ala Ser Ile
            340                 345                 350

Arg

<210>   30
<211>   273
<212>   PRT
<213>   Corynebacterium ammoniagenes

<400>   30
Met Thr Ala Pro Arg Asp Pro Phe Phe Pro Ala Asp Arg Ser Ile Arg
  1               5                  10                  15

Ala Ser Thr Ala Pro Val Glu Val Arg Arg Leu Gly Arg Met Asp Tyr
            20                  25                  30

Gln Glu Ala Trp Asp Tyr Gln Ala Glu Val Ala Ala Gln Arg Ala Arg
        35                  40                  45

Asp Glu Val Ala Asp Thr Leu Leu Val Val Glu His Pro Ala Val Tyr
    50                  55                  60

Thr Ala Gly Lys Arg Thr Gln Pro Glu Asp Met Pro Thr Asn Gly Leu
 65                  70                  75                  80

Pro Val Ile Asn Val Asp Arg Gly Gly Arg Ile Thr Trp His Gly Glu
                 85                  90                  95

Gly Gln Leu Val Val Tyr Pro Ile Ile Lys Leu Ala Glu Pro Val Asp
            100                 105                 110

Val Val Asp Tyr Val Arg Arg Leu Glu Glu Ala Val Ile His Thr Val
        115                 120                 125

Arg Glu Met Gly Val Thr Thr Ala Gly Arg Ile Asp Gly Arg Ser Gly
    130                 135                 140

Val Trp Val Pro Ser Thr Thr Ala Ala Lys Asp Pro Ala Ala Ser His
145                 150                 155                 160

Arg Asp Arg Lys Ile Ala Ala Leu Gly Ile Arg Ile Thr Arg Gly Val
                165                 170                 175

Thr Met His Gly Leu Ala Leu Asn Cys Asp Asn Ile Leu Asp Tyr Tyr
            180                 185                 190

Glu His Ile Ile Ala Cys Gly Ile Asp Asp Ala Asp Ile Thr Thr Leu
        195                 200                 205

Ala Leu Glu Leu Gly Arg Asp Val Thr Val Asp Asp Ala Val Glu Pro
    210                 215                 220

Leu Leu Ile Ala Leu Asp Asp Ala Leu Ala Gly Arg Met Val Val Ala
225                 230                 235                 240

Asp His Thr Phe Ala Ser Ala Pro Asp Pro Ile Lys Leu Ala Asn Glu
                245                 250                 255

Lys Ala Arg Gln Ala Arg Ala Gln Ser Ser Leu Thr Asp His Ala Gly
            260                 265                 270

Ser

<210> 31
<211> 2859
<212> DNA
<213> Corynebacterium ammoniagenes

<400> 31
atggatttca ttgcccgcca ccttgggcca gatgccacag aatctaagga catgctggcg 60

cgtgtgggtt atgacagcgt agaagcgctt gtcacctcag caatccccca gtccattagc 120

atcacggatg cgcttaatat gccgcaggca ttgagtgaga ccgacgcaca agccaagctt 180

cgcgcttacg ctgataaaaa tgtcgtgctg aagtctttct acggccaggg ctactcagac 240

accatcaccc ctgctgttat tcgccgcggt ttggtagaag acgctggttg gtacaccgct 300

tataccccat accagccaga aatttcccag ggtcgccttg agtcgctgct gaacttccag 360

accatggttc aagacctcac cggcttgcct attgcgaatg cttctttgct ggatgaggca 420

tcggcagttg ctgaggccgt gggtttgatg tctcgtgcgg tcaagaaggg ccgccgcgta 480

ctgctcgacg cccgtttgca cccacaggtt ctcaccgtgg cggcggagcg tgcccgagca 540

attgacctcg aagttgagat tgctgacttg agcaacggcg tggttggcga agacctcgtc 600

ggcgcagtag ttgcctacac cggtacggag ggcgatattt ttgacccacg tgctgttatc 660

gaagaaatcc atggccgcgg cggacttgtt tccgtcgcgg ctgacttgtt gtccttgctg 720

cttctggaag gcccaggctc gttcggtgca gacattgtca ttggttcctc ccaacgcttt 780

ggtgtgccgc tcttctttgg tggcccacac gctgctttca tggcagtaac tgacaagcta 840

aagcgtcaga tgccaggccg tttggtcggc gtatcggtcg attctgaggg ccgtcctgct 900

taccgcttgg cgctgcagac tcgtgaacag cacatccgcc gtgaacgcgc gacgtcaaat 960

atttgtaccg cgcaggcact tctggccaac gtggctgcca tgtacgccgt ctaccacggt 1020

ccagaaggct tgaaggagat tgctaaccac gtgcactcct tggctgcttc ctttgccggt 1080

gcagttacta ctcagggtct gaagattact tcctcggagt tcttcgacac cgttaccgtt 1140

gccggcgttg atgccgcatc cattaagttc agcttggaaa aggccggata cctggtgcgc 1200

accattggcg aggataaggt ttctgtctcc ttcggtgagt ccgcaaccca aggcgatgtt 1260

actgtcttgg cggacgcctt tggtgccgct gcagtagata atgcagattt cccactgcct 1320

gaagcactca cccgcaccac cgaggtgctc acccacgaaa tctttaactc cattcactcc 1380

gaaacccaga tgatgcgtta cctgcgcaag ctcggtgata aggatctggc tctagatcgc 1440

accatgattc ctttgggctc atgcaccatg aagctcaacc caaccgcagc catggaaccg 1500

atcacctggc cagaattcgc caatgttcac ccttactccc ctgaatacgc aacccagggc 1560

tggcgtgagc tcattgaaga gttggaaggc tggttggctg agctgaccgg ctacgccaag 1620

gtttctatcc aaccaaacgc tggttcccag ggcgagctag ctggtctttt ggctatccgc 1680

cgctaccacg tcgcaaatgg tgacaccaac cgcgatatcg tgttgattcc tgcgtccgcg 1740

cacggcacca acgctgcctc cgcgaccctg gcaaatctgc gcgttgttgt ggttaagacc 1800

gccgaagacg gctccatcga tctggaagat ctcgatgcga agatcgccaa gcatggtcag 1860

aacatggccg gaatcatgat cacctaccca tccactcacg gcgtctttga cccagaggtt 1920

cgtgaagtct gcgacaagat ccatgccgct ggcggccagg tctacattga tggcgcaaac 1980

atgaatgctt tgactggttg ggctcagccg ggcaagttcg gtggcgatgt ctcgcacttg 2040

aacctgcaca agactttcac cattccgcac ggcggtggcg gcccaggtgt tggaccaatt 2100

ggtgtcgctg agcacctcat tccattcctg ccaacggatg ctgcagctga tgagctggat 2160

cctgctaacc caaccccagt agaacagggc gttccaatta ctgcttcgca gtttggttcc 2220

gctggtgttc tgccgattac ctgggcatac atcgcaatga ccggtggcga gggtctaacc 2280

tccgctactg cacacgccat cttgggtgct aactaccttg cgcgcgaact ctccgattcc 2340

ttcccaattc tgttcaccgg taatgaaggt cttgttgcgc acgagtgcat tttggatctg 2400

cgcgcgctaa ccgatgcctc aggcgttact gcagcagacg ttgccaagcg tttgatcgac 2460

tttggcttcc acgctcctac cctcgcattc ccagtggctg gcaccttgat ggtggaacct 2520

actgagtctg aggatattgc tgaactggat cgtttcattg aagcaatgcg caccatccgt 2580

gcggagattc aggaaatcat cgatggcaag atcgcatatg aagattcggt catccgccac 2640

gcaccttaca ccgcaccgtc agtctcaagc gatgattggg agtactcctt tagccgtgaa 2700

aaggccgcat ggccagttcc ttcactgcgt ttgaacaagt acttcccacc ggtacgccgc 2760

ctggatgaag cttacggcga ccgcaacctg gtgtgctcct gcccaccgcc agaggcattc 2820

gacttcgatg ccgacaccga ttccaccgag gaggcttaa 2859

<210> 32
<211> 1104
<212> DNA
<213> Corynebacterium ammoniagenes

<400> 32
atgtcagaac tacgccagtc cccactgcac gcagagcacg aaaagctcgg cgcatccttt 60

accgcttttg gcccttggaa tatgccacta aagtacggca aggagctcga tgagcaccac 120

gcagtgcgta atgcagtcgg catgtttgac ctctcgcaca tgggtgagat ttgggtcaac 180

ggcccagacg ccgctgcatt tttgtcctat gcgctgatct ccaacatgga gaccgtgaaa 240

aatggcaagg cgaagtactc catgattgtt gctgaagacg gcggcatcat cgatgaccta 300

atttcctacc gtttctccga taccaagttc ttggtagtgc caaacgctgg caacactgat 360

gtggtttggg aagcttttaa tcagcgcatt gaaggcttcg atgtagaact caacaatgag 420

tccttggatg ttgcgatgat tgccctgcag ggccccaatg ctgccaaggt tctagttgaa 480

caggttgctg aagagtccaa ggaagaagta gaaaaccttc cttactatgc cgcaaccatg 540

gccaaagtcg cagacgttga caccatcgtc gcgcgcaccg gctacaccgg cgaagacggc 600

ttcgagctga tgatctacaa cgccgatgcg accaagctct ggcagctttt catcgaccaa 660

gatggtgtta ctccatgcgg tttagcttca cgcgattcct tgcgcttgga agctggcatg 720

cctttgtacg gcaatgagct ttcccgcgat atcacccctg tcgaggcagg catgggtgtg 780

gcgtttaaga agaagaccgc tgacttcgtc ggcgccgagg tcctgcgtca acgcttggaa 840

gaaggcccta agcaagttat caaggctttg acctcctctg agcgccgtgc agcgcgcacc 900

ggtgctgaaa tctatgccgg cgagcagttg gtaggcaccg taacttcggg tcagccatcg 960

ccgacgctgg gacaccctat tgccctggca ctggtagata ctgcagcaaa cctcgaagaa 1020

ggcgcagaag tagaagtgga tattcgcggc aagcgttacc ccttcaccgt taccaagacg 1080

cctttctata gccgcgagaa gtaa 1104

<210> 33
<211> 390
<212> DNA
<213> Corynebacterium ammoniagenes

<400> 33
atggctaacc tacctgcaga atttacttac tccgaagacc acgagtggat taacgccgct 60

caggacgcaa tcgttggcaa aactgttcgc atcggcatca cttctgttgc cgcagaccgt 120

cttggtgagg ttgtcttcgc tgagcttcca gcagttggcg atagcgtcac tgcaggtgaa 180

acctgtggtg aggttgaatc caccaagtcc gtttctgacc tgtacagccc tgtcaccggt 240

accgtgaccg ctgtgaacga gacagtgcac gatgattatg aaatcatcaa caatgatcct 300

ttcggtgaag gttggctgtt tgaggtcgag gttgaagaac tcggcgaggt tatgaccgct 360

gatgaatacg cggcagaaaa cggcatctaa 390

<210> 34
<211> 1062
<212> DNA
<213> Corynebacterium ammoniagenes

<400> 34
atgctccgca ttgaaaagaa gaatgcggag tcacccattg agcagaagcc gaggtggatc 60

cgcaaccagg tccgcactgg ccctggttat gaggacatga aaaagcgtgt tgctggcgct 120

ggcctgcaca ctgtctgcca ggaggcaggc tgtcctaata tccacgaatg ctgggagtcc 180

cgagaagcca ccttccttat cggcggtgac cgttgtactc gccgttgtga cttctgcgat 240

attgccactg gtaagccgca ggcattggac accgatgagc cgcgtcgcgt ttcggaaaac 300

atccaagaga tgaatctgaa ctacgccacg atcactggtg ttacccgtga tgaccttcca 360

gatgagggcg catggctata tgctgaagtg gttcgtaaga tccacgagaa gaaccctcac 420

acgggtgtag aaaacctcac cccggacttc tccggcaagc cagacctgct gcaggaagtc 480

ttcgaggctc gccctgaggt tttcgctcac aacttggaaa ccgttcctcg tattttcaag 540

cgtatccgcc cggcattccg ttatgagcgt tctctggatg ttttgcagca ggcacacgac 600

ttcggcctga tcaccaagtc gaacttgatc ttgggcatgg gtgaaactga ggaagagatt 660

caggaagctc tacgcgatat gcgctctgtg ggcactgaca tcattaccat tacgcagtac 720

ctgcgtcctg gtcctcgttt ccacccaatt gagcgttggg ttcgccctga ggagttcatt 780

gcgcactccg agtacgccaa ggaattgggc tttaccgtta tgtctggtcc tttggttcgt 840

tcttcttacc gcgcgggcaa gctctacacc caggcgatga aggcacgtgg ctgggagctg 900

ccagaaaatc tcaagcacct ggaagaaact tctgatggcg caaccgctca ggaagcttcg 960

tcgctgttga agaagtacgg cccttccgag gaaacgccag ttacttcccg catggcaaag 1020

acgcctgtgg gtgcagataa atttactgct agcatccgct aa 1062

<210> 35
<211> 822
<212> DNA
<213> Corynebacterium ammoniagenes

<400> 35
atgactgctc cgcgtgaccc gtttttcccc gctgatcgtt ctattcgcgc ttctactgcc 60

ccggtagagg tgagacgttt aggtcgtatg gattatcaag aagcctggga ctatcaagca 120

gaagtcgcag cgcagcgcgc acgtgacgaa gttgcagaca cgttgctggt cgttgagcac 180

cccgctgtgt atacggcggg caagcgcacg cagcccgaag atatgcccac caacggtctt 240

ccggttatca atgttgatcg tggcggccgg attacctggc acggcgaggg ccagttggtg 300

gtctacccga ttatcaagtt ggcagagcct gtcgatgtcg tcgattatgt ccgccgtctg 360

gaagaagctg ttattcatac cgttcgggaa atgggggtaa caactgctgg gcgtatcgat 420

ggtcgctcag gcgtgtgggt gccatcgact accgctgcga aagacccggc agcatcgcac 480

cgagaccgca agattgcggc cttaggcatc cgcattacgc gtggggttac catgcatggt 540

ctggcgctca attgcgacaa tatcttggac tactacgagc acattattgc ctgcggtatt 600

gatgatgccg atatcaccac cctggcgcta gagctgggcc gcgacgtcac cgtagatgat 660

gcggttgagc ccttgctcat tgcgcttgac gatgccttgg ccggccgcat ggtcgtcgcc 720

gaccacactt tcgcatctgc cccagacccc atcaaattag ctaatgagaa agcgcgccaa 780

gcacgcgcgc agtcttcctt gactgatcat gcaggctctt aa 822

<210>    36
<211>    40
<212>    DNA
<213>    Artificial Sequence

<220>
<223>    primer


<400>    36
gtacccgggg atcctctaga caaagccgaa gagaagttgg                              40


<210>    37
<211>    32
<212>    DNA
<213>    Artificial Sequence

<220>
<223>    primer


<400>    37
gcatagagta ctcggcgccc ataaatttca ct                                      32


<210>    38
<211>    32
<212>    DNA
<213>    Artificial Sequence

<220>
<223>    primer


<400>    38
gcgccgagta ctctatgccg agaagttgaa ca                                      32


<210>    39
<211>    40
<212>    DNA
<213>    Artificial Sequence

<220>
<223>    primer


<400>    39
gcctgcaggt cgactctaga ctgggcatca cactattttt                              40


<210>    40
<211>    531
<212>    DNA
<213>    Artificial Sequence

<220>
<223>    del-N2131L


<400>    40
gtacccgggg atcctctaga caaagccgaa gagaagttgg cttgacggac ccgaaattcc        60

agctgatttt gacgatcctg atgcacccgg caggtggcct ggcgaaaagt tggggcttcc 120

tcaagaaggg gccggctctc tgtcctcagt ggctcgtcgt atcggcgggg tctgcgtgga 180

ctggggtgtt tcctgggtta ttgctattgt gctgtccaat ttcacggatg tgctgggcga 240

tgtagcgaca tccacgctca ttattttcgt gatcctgggt tggcttaccg gttggatctt 300

tgctcgcacc ccaggtcatg ccgtgtttgg catgggcctt gcgcgtgtgg atgcagagga 360

acgtgtgggc tggtggcgtg cgctggttcg cccactgctg acgatcttga ttctgcctgc 420

cgtgatggtg gatgctgacg gccgtgggct ccacgacaag gcaacgggaa ctgcagttat 480

ccgcgggtaa tttgtcttga gtgaaattta tgggcgccga gtactctatg c 531

<210> 41
<211> 555
<212> DNA
<213> Artificial Sequence

<220>
<223> del-N2131R

<400> 41
gcgccgagta ctctatgccg agaagttgaa cagcaatctt gcagcaactc ctcagtattc 60

acaccagccc caatggacac aaaaacatca gccccagaat cgcccctaag ggcctcaaat 120

acacgatctg gaccaactag tcatcgggaa aacccaaccc cttaaatcgc cttctgcgct 180

taaggggtca atgctagata agtaggaaca acaacgtttg ggcggccagg atctttgcga 240

tcatcgccag cggatacaca gaggtataac ccatggcagg gagctcgttg cgggaggcat 300

ctgacacata actcagcaca gcagggtggg tttgcgtacc ggcgaggatg ccagcggttt 360

caccgaaggg gattttcatc agtttgtggc caacgaacag caccgtgatg gagatgaaca 420

aagtgagcag cgcaccgaag ccgatgatgg tgagtgattg ggggtcgctg atcgctgatc 480

gaaatcctgc gcccgctgag gtaccgatgg cagccaaaaa tagtgtgatg cccagtctag 540

agtcgacctg caggc 555

<210> 42
<211> 40
<212> DNA
<213> Artificial Sequence

<220>
<223> primer

<400> 42
gaaatttatg ggcgccgagt caggatgcaa ttgccatccg 40

<210> 43

<211>    32
<212>    DNA
<213>    Artificial Sequence

<220>
<223>    primer


<400>    43
gccattagcg tgttacttct cgcggctata ga                                    32


<210>    44
<211>    4499
<212>    DNA
<213>    Artificial Sequence

<220>
<223>    Pn_gcvPT(Cam)


<400>    44
gaaatttatg ggcgccgagt caggatgcaa ttgccatccg tcagatgtgc tacttgccat        60

ttacctacga ccaccaggtt gtagatggtg cagatgcagg tcgcttcatc accaccatca       120

aggaccgcct tgaaaccggt aacttcgaat ctgacttggc tctctaagtc cagtttcaga       180

atgtacggta gcttctagcc gtttgagtta gaagcaaaag ccaccatgta gaaatttcta       240

catggtggct ttgtggtttt ctcatctcta agttctcatt ttcgggggta taaaaaacta       300

acaaatccgc attgcagtct tcacattttc ttcaaatctg gcgttcatgc tggttataac       360

agcgctttcg caggagccat tacaaccggc ggctaggcgt gggtagaaaa gcaatgaaaa       420

gccggggcta gccatataat gaggaatgtt gactgccttc gtgcggtcgc tttagcaagc       480

ctggacaaat gtatcgttcc tccaaaggag tttgacactt atggatttca ttgcccgcca       540

ccttgggcca gatgccacag aatctaagga catgctggcg cgtgtgggtt atgacagcgt       600

agaagcgctt gtcacctcag caatccccca gtccattagc atcacggatg cgcttaatat       660

gccgcaggca ttgagtgaga ccgacgcaca agccaagctt cgcgcttacg ctgataaaaa       720

tgtcgtgctg aagtctttct acggccaggg ctactcagac accatcaccc ctgctgttat       780

tcgccgcggt ttggtagaag acgctggttg gtacaccgct tataccccat accagccaga       840

aatttcccag ggtcgccttg agtcgctgct gaacttccag accatggttc aagacctcac       900

cggcttgcct attgcgaatg cttctttgct ggatgaggca tcggcagttg ctgaggccgt       960

gggtttgatg tctcgtgcgg tcaagaaggg ccgccgcgta ctgctcgacg cccgtttgca      1020

cccacaggtt ctcaccgtgg cggcggagcg tgcccgagca attgacctcg aagttgagat      1080

tgctgacttg agcaacggcg tggttggcga agacctcgtc ggcgcagtag ttgcctacac      1140

cggtacggag ggcgatattt ttgacccacg tgctgttatc gaagaaatcc atggccgcgg      1200

cggacttgtt tccgtcgcgg ctgacttgtt gtccttgctg cttctggaag gcccaggctc      1260

gttcggtgca gacattgtca ttggttcctc ccaacgcttt ggtgtgccgc tcttctttgg 1320

tggcccacac gctgctttca tggcagtaac tgacaagcta aagcgtcaga tgccaggccg 1380

tttggtcggc gtatcggtcg attctgaggg ccgtcctgct taccgcttgg cgctgcagac 1440

tcgtgaacag cacatccgcc gtgaacgcgc gacgtcaaat atttgtaccg cgcaggcact 1500

tctggccaac gtggctgcca tgtacgccgt ctaccacggt ccagaaggct tgaaggagat 1560

tgctaaccac gtgcactcct tggctgcttc ctttgccggt gcagttacta ctcaggtctt 1620

gaagattact tcctcggagt tcttcgacac cgttaccgtt gccggcgttg atgccgcatc 1680

cattaagttc agcttggaaa aggccggata cctggtgcgc accattggcg aggataaggt 1740

ttctgtctcc ttcggtgagt ccgcaaccca aggcgatgtt actgtcttgg cggacgcctt 1800

tggtgccgct gcagtagata atgcagattt cccactgcct gaagcactca cccgcaccac 1860

cgaggtgctc acccacgaaa tctttaactc cattcactcc gaaacccaga tgatgcgtta 1920

cctgcgcaag ctcggtgata aggatctggc tctagatcgc accatgattc ctttgggctc 1980

atgcaccatg aagctcaacc caaccgcagc catggaaccg atcacctggc cagaattcgc 2040

caatgttcac ccttactccc ctgaatacgc aacccagggc tggcgtgagc tcattgaaga 2100

gttggaaggc tggttggctg agctgaccgg ctacgccaag gtttctatcc aaccaaacgc 2160

tggttcccag ggcgagctag ctggtctttt ggctatccgc cgctaccacg tcgcaaatgg 2220

tgacaccaac cgcgatatcg tgttgattcc tgcgtccgcg cacggcacca acgctgcctc 2280

cgcgaccctg gcaaatctgc gcgttgttgt ggttaagacc gccgaagacg gctccatcga 2340

tctggaagat ctcgatgcga agatcgccaa gcatggtcag aacatggccg gaatcatgat 2400

cacctaccca tccactcacg gcgtctttga cccagaggtt cgtgaagtct gcgacaagat 2460

ccatgccgct ggcggccagg tctacattga tggcgcaaac atgaatgctt tgactggttg 2520

ggctcagccg ggcaagttcg gtggcgatgt ctcgcacttg aacctgcaca agactttcac 2580

cattccgcac ggcggtggcg gcccaggtgt tggaccaatt ggtgtcgctg agcacctcat 2640

tccattcctg ccaacggatg ctgcagctga tgagctggat cctgctaacc caaccccagt 2700

agaacagggc gttccaatta ctgcttcgca gtttggttcc gctggtgttc tgccgattac 2760

ctgggcatac atcgcaatga ccggtggcga gggtctaacc tccgctactg cacacgccat 2820

cttgggtgct aactaccttg cgcgcgaact ctccgattcc ttcccaattc tgttcaccgg 2880

taatgaaggt cttgttgcgc acgagtgcat tttggatctg cgcgcgctaa ccgatgcctc 2940

aggcgttact gcagcagacg ttgccaagcg tttgatcgac tttggcttcc acgctcctac 3000

cctcgcattc ccagtggctg gcaccttgat ggtggaacct actgagtctg aggatattgc 3060

tgaactggat cgtttcattg aagcaatgcg caccatccgt gcggagattc aggaaatcat 3120

cgatggcaag atcgcatatg aagattcggt catccgccac gcaccttaca ccgcaccgtc 3180

agtctcaagc gatgattggg agtactcctt tagccgtgaa aaggccgcat ggccagttcc 3240

ttcactgcgt ttgaacaagt acttcccacc ggtacgccgc ctggatgaag cttacggcga 3300

ccgcaacctg gtgtgctcct gcccaccgcc agaggcattc gacttcgatg ccgacaccga 3360

ttccaccgag gaggcttaaa tcaatgtcag aactacgcca gtccccactg cacgcagagc 3420

acgaaaagct cggcgcatcc tttaccgctt ttggcccttg gaatatgcca ctaaagtacg 3480

gcaaggagct cgatgagcac cacgcagtgc gtaatgcagt cggcatgttt gacctctcgc 3540

acatgggtga gatttgggtc aacggcccag acgccgctgc atttttgtcc tatgcgctga 3600

tctccaacat ggagaccgtg aaaaatggca aggcgaagta ctccatgatt gttgctgaag 3660

acggcggcat catcgatgac ctaatttcct accgtttctc cgataccaag ttcttggtag 3720

tgccaaacgc tggcaacact gatgtggttt gggaagcttt taatcagcgc attgaaggct 3780

tcgatgtaga actcaacaat gagtccttgg atgttgcgat gattgccctg cagggcccca 3840

atgctgccaa ggttctagtt gaacaggttg ctgaagagtc caaggaagaa gtagaaaacc 3900

ttccttacta tgccgcaacc atggccaaag tcgcagacgt tgacaccatc gtcgcgcgca 3960

ccggctacac cggcgaagac ggcttcgagc tgatgatcta caacgccgat gcgaccaagc 4020

tctggcagct tttcatcgac caagatggtg ttactccatg cggtttagct tcacgcgatt 4080

ccttgcgctt ggaagctggc atgcctttgt acggcaatga gctttcccgc gatatcaccc 4140

ctgtcgaggc aggcatgggt gtggcgttta agaagaagac cgctgacttc gtcggcgccg 4200

aggtcctgcg tcaacgcttg gaagaaggcc ctaagcaagt tatcaaggct ttgacctcct 4260

ctgagcgccg tgcagcgcgc accggtgctg aaatctatgc cggcgagcag ttggtaggca 4320

ccgtaacttc gggtcagcca tcgccgacgc tgggacaccc tattgccctg gcactggtag 4380

atactgcagc aaacctcgaa gaaggcgcag aagtagaagt ggatattcgc ggcaagcgtt 4440

accccttcac cgttaccaag acgcctttct atagccgcga gaagtaacac gctaatggc 4499

<210> 45
<211> 32
<212> DNA
<213> Artificial Sequence

<220>
<223> primer

<400> 45
cgcgagaagt aacacgctaa tggcgcattg aa 32

<210> 46
<211> 40
<212> DNA

<213>    Artificial Sequence

<220>
<223>    primer

<400>    46
tcaacttctc ggcatagagt ttagcggatg ctagcagtaa                              40

<210>    47
<211>    3053
<212>    DNA
<213>    Artificial Sequence

<220>
<223>    Pn_gcvH-lipBA(Cam)

<400>    47
cgcgagaagt aacacgctaa tggcgcattg aatcgccggc cgatatttca gggcttgtac        60

gaaaacggca tcatcgaagc agatggcacc caagttgaag tcgacactat catttgggcg       120

attggctttc gtcccagcct ggcgcacttg gacccgctga acctttataa cgagaagggc       180

gggatcaaca ttgagggcac aaaggtaaag ggaaagaaga atcttcacct aattggctat       240

ggcccttccc aatccacagt aggagctaac cgggcaggtc gagctgccgt catcagcatt       300

gcccgcgacc tgaaatctgg cacctaggca ctgctcaatg tccgatggga gcaaactctt       360

aacccccgaa ggggcaatat caacgtgcga tagtggcatg taaagtgctt cgctgcgcta       420

aagtgaaagc gatacttcaa cccatgcacc tattcgctgc attcccggcg gatcaggcac       480

gtcaatgaaa ggatagttcc tatggctaac ctacctgcag aatttactta ctccgaagac       540

cacgagtgga ttaacgccgc tcaggacgca atcgttggca aaactgttcg catcggcatc       600

acttctgttg ccgcagaccg tcttggtgag gttgtcttcg ctgagcttcc agcagttggc       660

gatagcgtca ctgcaggtga aacctgtggt gaggttgaat ccaccaagtc cgtttctgac       720

ctgtacagcc ctgtcaccgg taccgtgacc gctgtgaacg agacagtgca cgatgattat       780

gaaatcatca acaatgatcc tttcggtgaa ggttggctgt ttgaggtcga ggttgaagaa       840

ctcggcgagg ttatgaccgc tgatgaatac gcggcagaaa acggcatcta agcctttttt       900

acttttcaca cctttaagcg cggagcagtt tgccccaagt actacactgg gaaaacatga       960

ctgctccgcg tgacccgttt ttccccgctg atcgttctat tcgcgcttct actgccccgg      1020

tagaggtgag acgtttaggt cgtatggatt atcaagaagc ctgggactat caagcagaag      1080

tcgcagcgca gcgcgcacgt gacgaagttg cagacacgtt gctggtcgtt gagcaccccg      1140

ctgtgtatac ggcgggcaag cgcacgcagc ccgaagatat gcccaccaac ggtcttccgg      1200

ttatcaatgt tgatcgtggc ggccggatta cctggcacgg cgagggccag ttggtggtct      1260

acccgattat caagttggca gagcctgtcg atgtcgtcga ttatgtccgc cgtctggaag      1320

aagctgttat tcataccgtt cgggaaatgg gggtaacaac tgctgggcgt atcgatggtc 1380

gctcaggcgt gtgggtgcca tcgactaccg ctgcgaaaga cccggcagca tcgcaccgag 1440

accgcaagat tgcggcctta ggcatccgca ttacgcgtgg ggttaccatg catggtctgg 1500

cgctcaattg cgacaatatc ttggactact acgagcacat tattgcctgc ggtattgatg 1560

atgccgatat caccaccctg gcgctagagc tgggccgcga cgtcaccgta gatgatgcgg 1620

ttgagccctt gctcattgcg cttgacgatg ccttggccgg ccgcatggtc gtcgccgacc 1680

acactttcgc atctgcccca gaccccatca aattagctaa tgagaaagcg cgccaagcac 1740

gcgcgcagtc ttccttgact gatcatgcag gctcttaagt agtgccccct attcgggcag 1800

cgatgtccat tcttggaata tttcaggaac cggtgggatt gaaggaatta aggccttaag 1860

cgcacccatc aacggggtca aaaatacgag gtcagtatca taattagttg aatttaaaat 1920

cacacaacac gtagggtgga atttgtgact attaagcctg aaggacgcaa gatgctccgc 1980

attgaaaaga agaatgcgga gtcacccatt gagcagaagc cgaggtggat ccgcaaccag 2040

gtccgcactg gccctggtta tgaggacatg aaaaagcgtg ttgctggcgc tggcctgcac 2100

actgtctgcc aggaggcagg ctgtcctaat atccacgaat gctgggagtc ccgagaagcc 2160

accttcctta tcggcggtga ccgttgtact cgccgttgtg acttctgcga tattgccact 2220

ggtaagccgc aggcattgga caccgatgag ccgcgtcgcg tttcggaaaa catccaagag 2280

atgaatctga actacgccac gatcactggt gttacccgtg atgaccttcc agatgagggc 2340

gcatggctat atgctgaagt ggttcgtaag atccacgaga agaaccctca cacgggtgta 2400

gaaaacctca ccccggactt ctccggcaag ccagacctgc tgcaggaagt cttcgaggct 2460

cgccctgagg ttttcgctca caacttggaa accgttcctc gtattttcaa gcgtatccgc 2520

ccggcattcc gttatgagcg ttctctggat gttttgcagc aggcacacga cttcggcctg 2580

atcaccaagt cgaacttgat cttgggcatg ggtgaaactg aggaagagat tcaggaagct 2640

ctacgcgata tgcgctctgt gggcactgac atcattacca ttacgcagta cctgcgtcct 2700

ggtcctcgtt tccacccaat tgagcgttgg gttcgccctg aggagttcat tgcgcactcc 2760

gagtacgcca aggaattggg ctttaccgtt atgtctggtc ctttggttcg ttcttcttac 2820

cgcgcgggca agctctacac ccaggcgatg aaggcacgtg gctgggagct gccagaaaat 2880

ctcaagcacc tggaagaaac ttctgatggc gcaaccgctc aggaagcttc gtcgctgttg 2940

aagaagtacg gcccttccga ggaaacgcca gttacttccc gcatggcaaa gacgcctgtg 3000

ggtgcagata aatttactgc tagcatccgc taaactctat gccgagaagt tga 3053

<210> 48
<211> 281
<212> PRT

<213>    Artificial Sequence

<220>
<223>    C.gl HisG variant


<400>    48
Met Leu Lys Ile Ala Val Pro Asn Lys Gly Ser Leu Ser Glu Arg Ala
1               5                   10                  15

Met Glu Ile Leu Ala Glu Ala Gly Tyr Ala Gly Arg Gly Asp Ser Lys
            20                  25                  30

Ser Leu Asn Val Phe Asp Glu Ala Asn Asn Val Glu Phe Phe Phe Leu
        35                  40                  45

Arg Pro Lys Asp Ile Ala Ile Tyr Val Ala Gly Gly Gln Leu Asp Leu
    50                  55                  60

Gly Ile Thr Gly Arg Asp Leu Ala Arg Asp Ser Gln Ala Asp Val His
65                  70                  75                  80

Glu Val Leu Ser Leu Gly Phe Gly Ser Ser Thr Phe Arg Tyr Ala Ala
                85                  90                  95

Pro Ala Asp Glu Glu Trp Ser Ile Glu Lys Leu Asp Gly Lys Arg Ile
            100                 105                 110

Ala Thr Ser Tyr Pro Asn Leu Val Arg Asp Asp Leu Ala Ala Arg Gly
        115                 120                 125

Leu Ser Ala Glu Val Leu Arg Leu Asp Gly Ala Val Glu Val Ser Ile
    130                 135                 140

Lys Leu Gly Val Ala Asp Ala Ile Ala Asp Val Val Ser Thr Gly Arg
145                 150                 155                 160

Thr Leu Arg Gln Gln Gly Leu Ala Pro Phe Gly Glu Val Leu Cys Thr
                165                 170                 175

Ser Glu Ala Val Ile Val Gly Arg Lys Asp Glu Lys Val Thr Pro Glu
            180                 185                 190

Gln Gln Ile Leu Leu Arg Arg Ile Gln Gly Ile Leu His Ala Gln Asn
        195                 200                 205

Phe Leu Met Leu Asp Tyr Asn Val Asp Arg Asp Asn Leu Asp Ala Ala
    210                 215                 220

Thr Ala Val Thr Pro Gly Leu Ser His Pro Gln Val Ser Pro Leu Ala
225                 230                 235                 240

Arg Asp Asn Trp Val Ala Val Arg Ala Met Val Pro Arg Arg Ser Ala
                245                 250                 255

Asn Ala Ile Met Asp Lys Leu Ala Gly Leu Gly Ala Glu Ala Ile Leu
            260                 265                 270

Ala Ser Glu Ile Arg Ile Ala Arg Ile
        275                 280


<210>    49

<211>    35
<212>    DNA
<213>    Artificial Sequence

<220>
<223>    primer


<400>    49
tcgagctcgg tacccatcgc catctacgtt gctgg                                35


<210>    50
<211>    35
<212>    DNA
<213>    Artificial Sequence

<220>
<223>    primer


<400>    50
tcgagctcgg tacccatcgc catctacgtt gctgg                                35


<210>    51
<211>    45
<212>    DNA
<213>    Artificial Sequence

<220>
<223>    primer


<400>    51
gtgccagtgg ggatacctgt gggtgggata agcctggggt tactg                     45


<210>    52
<211>    45
<212>    DNA
<213>    Artificial Sequence

<220>
<223>    primer


<400>    52
aaccccaggc ttatcccacc cacaggtatc cccactggca cgcga                     45


<210>    53
<211>    35
<212>    DNA
<213>    Artificial Sequence

<220>
<223>    primer


<400>    53
ctctagagga tccccgggac gtggttgatg gtggt                                35

<210> 54
<211> 35
<212> DNA
<213> Artificial Sequence

<220>
<223> primer

<400> 54
tcgagctcgg tacccatcgc catctacgtt gctgg 35

<210> 55
<211> 35
<212> DNA
<213> Artificial Sequence

<220>
<223> primer

<400> 55
gagtctagaa gtactcgaga tgctgacctc gtttc 35

<210> 56
<211> 35
<212> DNA
<213> Artificial Sequence

<220>
<223> primer

<400> 56
agcatctcga gtacttctag actcgcacga aaaag 35

<210> 57
<211> 35
<212> DNA
<213> Artificial Sequence

<220>
<223> primer

<400> 57
ctctagagga tcccctttgg gcagagctca aattc 35

<210> 58
<211> 40
<212> DNA
<213> Artificial Sequence

<220>
<223> primer

<400> 58

gcatgggctt gctgctcgag ttcagggtag ttgactaaag                                40

<210>    59
<211>    20
<212>    DNA
<213>    Artificial Sequence

<220>
<223>    primer

<400>    59
agtttcgtaa cccaccttgc                                                      20

<210>    60
<211>    20
<212>    DNA
<213>    Artificial Sequence

<220>
<223>    primer

<400>    60
cgcttctcaa tctgatgaga                                                      20

<210>    61
<211>    35
<212>    DNA
<213>    Artificial Sequence

<220>
<223>    primer

<400>    61
gtcagcatct cgagtgctcc ttagggagcc atctt                                     35

<210>    62
<211>    35
<212>    DNA
<213>    Artificial Sequence

<220>
<223>    primer

<400>    62
gtcaaatgtc ttcacatgtg tgcacctttc gatct                                     35

<210>    63
<211>    35
<212>    DNA
<213>    Artificial Sequence

<220>
<223>    primer

<400>    63
gaaaggtgca cacatgtgaa gacatttgac tcgct                                35

<210>    64
<211>    35
<212>    DNA
<213>    Artificial Sequence

<220>
<223>    primer

<400>    64
tcgtttttag gcctcctaga tgcgggcgat gcgga                                35

<210>    65
<211>    35
<212>    DNA
<213>    Artificial Sequence

<220>
<223>    primer

<400>    65
atcgcccgca tctaggaggc ctaaaaacga ccgag                                35

<210>    66
<211>    35
<212>    DNA
<213>    Artificial Sequence

<220>
<223>    primer

<400>    66
gacagttttg gtcatgttgt gtctcctcta aagat                                35

<210>    67
<211>    35
<212>    DNA
<213>    Artificial Sequence

<220>
<223>    primer

<400>    67
tagaggagac acaacatgac caaaactgtc gccct                                35

<210>    68
<211>    35
<212>    DNA
<213>    Artificial Sequence

<220>

<223>    primer

<400>    68
tgaagcgccg gtaccgctta cagcaaaacg tcatt                                35

<210>    69
<211>    35
<212>    DNA
<213>    Artificial Sequence

<220>
<223>    primer

<400>    69
cgttttgctg taagcggtac cggcgcttca tgtca                                35

<210>    70
<211>    35
<212>    DNA
<213>    Artificial Sequence

<220>
<223>    primer

<400>    70
agtgacattc aacattgttt tgatctcctc caata                                35

<210>    71
<211>    35
<212>    DNA
<213>    Artificial Sequence

<220>
<223>    primer

<400>    71
gaggagatca aaacaatgtt gaatgtcact gacct                                35

<210>    72
<211>    35
<212>    DNA
<213>    Artificial Sequence

<220>
<223>    primer

<400>    72
cgctgggatg tttctctaga gcgctccctt agtgg                                35

<210>    73
<211>    35
<212>    DNA
<213>    Artificial Sequence

<220>
<223>    primer


<400>    73
aagggagcgc tctagagaaa catcccagcg ctact                                35


<210>    74
<211>    35
<212>    DNA
<213>    Artificial Sequence

<220>
<223>    primer


<400>    74
agtcatgcct tccatgagtg tttcctttcg ttggg                                35


<210>    75
<211>    35
<212>    DNA
<213>    Artificial Sequence

<220>
<223>    primer


<400>    75
cgaaaggaaa cactcatgga aggcatgact aatcc                                35


<210>    76
<211>    35
<212>    DNA
<213>    Artificial Sequence

<220>
<223>    primer


<400>    76
cgagtctaga agtgcctatt ttaaacgatc cagcg                                35